# EUROPEAN PATENT APPLICATION

(11) **EP 4 821 423 A2**
(43) Date of publication of application: **14.10.2026**
(21) Application number: 26201806.2
(22) Date of filing: 28.08.2014
(51) Int. Cl.: A61B 1/31

(54) **COLON CLEANING SYSTEM WITH AUTOMATIC SELF-PURGING FEATURES**

(30) Priority: 29.08.2013 US 201361871463 P
(62) Document number(s) of the earlier application(s) in accordance with Art. 76 EPC: 23174673.6 / 4 233 986; 14771962.9 / 3 038 668
(71) Applicant: Motus GI Medical Technologies Ltd., 3902638 Tirat HaCarmel (IL)
(72) Inventor: HASSIDOV, Noam, 2521300 Doar-Na Oshrat (IL); KOCHAVI, Eyal, 3452620 Haifa (IL); TSUKRAN, Roi, 7575721 Rishon-LeZion (IL); SHTUL, Boris, 3085000 Doar-Na Hof HaCarmel (IL); LULEKO, Kobi, 2019600 Eshchar (IL); ARNON, Tzach, 2018000 Doar-Na Misgav (IL)
(74) Representative: Mathys & Squire

(57) **Abstract**

Systems and methods for cleaning a colon or other portion of an intestine include optional use of sensors to detect conditions of blockage of flow of materials within an evacuation channel used to remove fecal material from the body; and devices and methods for purging such blockages from the evacuation channel.

## Description

### FIELD AND BACKGROUND OF THE INVENTION

The present invention, in some embodiments thereof, relates to a system for cleaning a colon or other body lumen, and, more particularly but not exclusively, to systems and methods for cleaning comprising automated self-cleaning features.

Current gastrointestinal technology includes various methods for cleaning fecal matter from a colon. In cases of impacted feces following long constipation, colon cleaning itself can be a goal of a procedure. In other cases, colon cleaning is necessary or desirable to enable unimpeded inspection of the colon tissues and/or to facilitate diagnostic examinations and/or treatment of those tissues.

A colonoscope provides means for optically and/or electronically imaging the colon and its contents. In some methods of colon observation, imaging occurs while flushing or washing a portion of the colon with an irrigating fluid. Irrigating fluid, fecal matter and/or other colon contents are drawn out of the colon by suction and/or other methods for transporting matter out of the body.

In some cases, a working channel of a colonoscope provides irrigation to the colon. In some cases, a working channel of a colonoscope transports fecal materials from the body. In some cases, irrigation and/or suction channels are connectable to the colonoscope for coordinated use of a cleaning system and colonoscope. Cleaning systems with features intended to purge developed blockages which cause problems by preventing evacuation flow are known in the art.

The following patent applications pertain to the field of endeavor of the current application: U.S. Patent Application 2010/0185056 by Tal Gordon et al.; U.S. Patent Application 2011/0105845 by Tal Gordon et al.; and U.S. Patent Application 2012/0101336 by Yoav Hirsch et al.

### SUMMARY OF THE INVENTION

According to an aspect of some embodiments of the present invention, there is provided a system for cleaning an intestine, comprising: an evacuation lumen for evacuating irrigation fluid from the intestine; a variable output pressure source; at least one sensor, positioned to detect an environmental condition in or near the evacuation lumen; and a controller configured to: determine a developing blockage of the evacuation lumen based on reporting from the at least one sensor, and vary the pressure from the pressure source for purging of the developing blockage based on the determination.

According to some embodiments of the invention, the pressure source is operable to alternately apply proximally-directed and distally-directed pressure gradients to the evacuation lumen.

According to some embodiments of the invention, the evacuation lumen is at least as long as a human colon.

According to some embodiments of the invention, the developing blockage is determined while the rate of evacuation matches or exceeds the rate of introduction of the irrigation fluid to the intestine.

According to some embodiments of the invention, the developing blockage comprises less than a 50% occlusion of a cross-section of an evacuation lumen.

According to some embodiments of the invention, the developing blockage comprises less than a 10% occlusion of a cross-section of an evacuation lumen.

According to some embodiments of the invention, the developing blockage comprises an onset of a decreasing rate of evacuation due to increasing obstruction of an evacuation lumen.

According to some embodiments of the invention, the developing blockage comprises a blockage detected within 100 msec of reaching an occlusion of more than 50% of an evacuation lumen.

According to some embodiments of the invention, the developing blockage comprises a blockage detected within 50 msec of reaching an occlusion of more than 80% of an evacuation lumen.

According to some embodiments of the invention, the varying of pressure is configured to comprise at least two cycles of increasing and then decreasing the distally directed pressure.

According to some embodiments of the invention, the controller is configured to redetermine blockage during the varying of pressure.

According to some embodiments of the invention, the controller is configured to adjust the varying of pressure based upon the finding of the redetermination being an unchanged blockage.

According to some embodiments of the invention, the adjustment comprises a termination of pressure variation.

According to some embodiments of the invention, the adjustment comprises an increasing of pressure variation.

According to some embodiments of the invention, the determination comprises determination of a location in the system of a developing blockage, the determination of a location being a basis for the variation of pressure.

According to an aspect of some embodiments of the present invention, there is provided the system of claim 1, with at least two of the at least one sensor selected from a group consisting of: a) a sensor positioned on external surface of a distal portion of the evacuation lumen and external to the lumen; b) a sensor positioned within the evacuation lumen and within 5 mm of a distal end of the evacuation lumen; c) a sensor positioned within the evacuation lumen and 5-30 mm of a distal end of the evacuation lumen; d) a sensor positioned within the evacuation lumen and 3-160 cm from a distal end of the evacuation lumen; e) a sensor positioned within the evacuation lumen and 160-250 cm from a distal end of the evacuation lumen; f) a sensor positioned within the evacuation lumen and at least 250 cm from a distal end of the evacuation lumen; and g) a sensor positioned within a fluid supply tube which supplies a fluid flow to a distal portion of the evacuation lumen.

According to some embodiments of the invention, the pressure source comprises a first pump operable to pump material distally through the evacuation lumen.

According to some embodiments of the invention, the first pump is also operable to pump material proximally through the evacuation lumen.

According to some embodiments of the invention, the pressure source comprises a second pump operable to pump material proximally through the evacuation lumen.

According to some embodiments of the invention, the pressure source comprises a valve and vacuum source inlet, the valve being controllable for connecting and disconnecting the evacuation lumen with the vacuum source inlet.

According to some embodiments of the invention, the pressure source comprises a valve and a pressurized fluid inlet, the valve being controllable for connecting and disconnecting the evacuation lumen with the pressurized fluid inlet.

According to some embodiments of the invention, the at least one sensor comprises an optical sensor.

According to some embodiments of the invention, the optical sensor senses at least one of the group consisting of a turbidity, a particle size, a particle number, or a particle spectrum.

According to some embodiments of the invention, the at least one sensor comprises a sensor for sensing a bulk material property.

According to some embodiments of the invention, the bulk material property is at least one of the group consisting of a pH, a conductance, a solute concentration, or an osmotic pressure.

According to some embodiments of the invention, the at least one sensor comprises a flow sensor.

According to some embodiments of the invention, the at least one sensor comprises a fluid pressure sensor.

According to some embodiments of the invention, the determination of a developing blockage comprises at least one of the group consisting of a change in a sensed: a) pressure from one or more sensors; b) pressure difference among two or more sensors; c) flow; d) optical property; and e) bulk material property.

According to some embodiments of the invention, the system comprises a plurality of evacuation lumens.

According to some embodiments of the invention, the pressure applied to each of the plurality of evacuation lumens by the pressure source is individually controllable.

According to some embodiments of the invention, the evacuation pressure in a first of the plurality of evacuation lumens is distally directable, simultaneously with the evacuation pressure in a second of the plurality of evacuation lumens being proximally directed.

According to some embodiments of the invention, the plurality of evacuation lumens comprises a joining connection within 100 cm of the distal end of the lumens, sized for the passage of evacuated material.

According to some embodiments of the invention, the at least one sensor is positioned to detect pressure in the evacuation lumen; and the determination of a developing blockage comprises determination of a pressure change in the evacuation lumen.

According to some embodiments of the invention, the determination of a pressure change comprises determination of an onset of a pressure change.

According to some embodiments of the invention, the determination of a pressure change comprises determination of a change of at least 10 mmHg pressure within 100 msec.

According to some embodiments of the invention, the determination of a pressure change comprises determination of a change of at least 20 mmHg pressure within 150 msec.

According to some embodiments of the invention, the determination of a pressure change comprises determination of a change of at least 30 mmHg pressure within 200 msec.

According to some embodiments of the invention, the determination of a pressure change comprises determination of a differential change between two of the at least one sensor of at least 10 mmHg pressure within 100 msec.

According to an aspect of some embodiments of the present invention, there is provided a method for purging blockage of an evacuation lumen of an intestinal cleaning system, comprising: inserting an evacuation lumen at least 50 cm into a colon; evacuating waste from a colon proximally through the evacuation lumen; automatically detecting a developing blockage of the evacuation lumen; and initiating variation of the pressure in the evacuation lumen based on the automatic detection of a blockage.

According to some embodiments of the invention, the inserting is to within 20 cm of the distal end of the colon.

According to some embodiments of the invention, the method comprises: automatically determining that the developing blockage has been reduced; and stopping the automatic pressure variation.

According to some embodiments of the invention, the pressure variation comprises alternating the pressure in the evacuation lumen between distally-directed and proximally-directed pressures.

According to some embodiments of the invention, the pressure variation is at a frequency above 1 Hz.

According to some embodiments of the invention, the pressure variation reverses the pressure between distally-directed and proximally-directed at a frequency above 1 Hz.

According to some embodiments of the invention, the pressure variation is at a frequency above 5 Hz.

According to some embodiments of the invention, the pressure variation reverses the pressure from distally-directed to proximally-directed at a frequency above 5 Hz.

According to some embodiments of the invention, the automatic detection is performed at least 5 times per minute.

According to some embodiments of the invention, the evacuation lumen comprises a plurality of evacuation sub-lumens arranged alongside each other, and the variation of pressure is controlled so that a distally-directed pressure is applied to one sub-lumen while a proximally-directed pressure is applied to another sub-lumen.

According to some embodiments of the invention, the plurality of sub-lumens are joined by a connection allowing fluid communication, the join being within 20 mm of the distal end of the sub-lumens.

According to some embodiments of the invention, the initiated variation of pressure is modulated based on the determined degree of developing blockage.

According to some embodiments of the invention, the initiated variation of pressure is modulated based on the determined location of developing blockage.

According to some embodiments of the invention, the detecting of a developing blockage comprises a sensed reduction of flow in the evacuation lumen.

According to some embodiments of the invention, the detecting of a developing blockage comprises a sensed pressure change in the evacuation lumen.

According to some embodiments of the invention, the developing blockage detected is associated with a determined blockage location, and variation of pressure is modulated based on the blockage location.

According to some embodiments of the invention, the method comprises changing a pressure of irrigation fluid supplied to the colon at the distal end of the evacuation lumen.

According to some embodiments of the invention, the supplied irrigation fluid washes the distal end of the evacuation lumen.

According to an aspect of some embodiments of the present invention, there is provided a system for cleaning an intestine, comprising: a plurality of evacuation lumens for evacuating irrigation fluid from the intestine; a pressure source operable to individually apply alternately proximally-directed and distally-directed pressure gradients to each of the plurality of evacuation lumens; at least one sensor, positioned to detect an environmental condition in or near the plurality of evacuation lumens; at least one of the at least one sensor being configured to report a level of intracolonic pressure; and a controller configured to: determine the presence of blockage of the evacuation lumen based on reporting from the at least one sensor, determine if the level of intracolonic pressure satisfies a pressure safety condition, and vary the pressure from the pressure source for purging of the blockage based on the blockage presence only if the pressure safety condition is met.

According to some embodiments of the invention, the pressure safety condition is met by an intracolonic pressure of less than 150 mmHg.

According to some embodiments of the invention, the pressure safety condition is met by an intracolonic pressure of less than 200 mmHg.

According to an aspect of some embodiments of the present invention, there is provided a system for cleaning an intestine, comprising: an evacuation lumen for evacuating irrigation fluid from the intestine; a pressure source operable to alternately apply proximally-directed and distally-directed pressure gradients to the evacuation lumen; at least one sensor, positioned to detect an environmental condition in or near the evacuation lumen; and a controller configured to: determine a flow restriction within the evacuation lumen based on reporting from the at least one sensor, and vary the pressure from the pressure source for reduction of the flow restriction based on the determination.

According to some embodiments of the invention, the flow restriction comprises a partial lining of material accumulated on the wall of the evacuation lumen.

According to some embodiments of the invention, the flow restriction comprises a change in the shape of the evacuation lumen.

According to some embodiments of the invention, the flow restriction comprises turbulence in flow through the evacuation lumen.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

As will be appreciated by one skilled in the art, aspects of the present invention may be embodied as a system, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer readable program code embodied thereon. Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber cable, RF, etc., or any suitable combination of the foregoing.

Computer program code for carrying out operations for aspects of the present invention may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described below with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 schematically illustrates an exemplary colon cleaning system comprising a colonoscope workstation and a cleansing workstation, according to some embodiments of the present invention;
FIGs. 2A-2D schematically illustrate sensors and sensing modules of an exemplary colon cleaning system, according to some embodiments of the present invention;
FIGs. 3A-3E schematically illustrate sensors and sensing modules of an exemplary colon cleaning system, according to some embodiments of the present invention;
FIGs. 4A-4E schematically illustrate a cleaning system wherein flow of material through the system is blocked by clogs, according to some embodiments of the present invention;
FIG. 4F schematically illustrates a cleaning system, with tables of exemplary sensor readings from sensors within a cleaning system, according to some embodiments of the present invention;
FIGs. 5A-5C schematically illustrate a cleaning system comprising a bi-directional pump, wherein flow of material through the system is blocked by clogs and then purged, according to some embodiments of the present invention;
FIG. 6 is a simplified flow chart of a colon cleaning method; according to some embodiments of the present invention;
FIG. 7A schematically illustrates an exemplary colon cleaning system comprising a colonoscope workstation and a cleansing workstation, according to some embodiments of the present invention;
FIG. 7B schematically illustrates a magnified view of a colonoscope tip in proximity to colon content, according to some embodiments of the present invention;
FIG. 8A schematically illustrates a cleaning system comprising two bi-directional pumps, each for a separate evacuation line, according to some embodiments of the present invention;
FIGs. 8B-8E are exemplary plots of relative pressure versus time in a pair of colon cleaning system evacuation channels, according to some embodiments of the present invention;
FIG. 8F is a schematic illustration of an inter-evacuation channel fluid bridge, according to some exemplary embodiments of the invention;
FIG. 9A schematically illustrates an exemplary cleansing working station with a disposable tube assembly comprising a water and/or air pipe for irrigation, and an evacuation pipe, according to some embodiments of the present invention;
FIG. 9B schematically illustrates an exemplary cleansing working station having a disposable tube assembly comprising air and water pipes for irrigation and for dual-channel evacuation, according to some embodiments of the present invention;
FIG. 10 schematically illustrates a colon working station together with a cleansing working station and a disposable apparatus, according to some embodiments of the present invention; and
FIG. 11 schematically illustrates a cleaning system comprising two bi-directional pumps together with a colonoscope, according to some embodiments of the present invention.

### DESCRIPTION OF SPECIFIC EMBODIMENTS OF THE INVENTION

The present invention, in some embodiments thereof, relates to a system for cleaning a colon or other body lumen, and, more particularly but not exclusively, to systems and methods for cleaning comprising automated self-cleaning features.

### Overview

A broad aspect of some embodiments of the invention relates to tools and techniques for purging an evacuation channel of a cleaning system from fecal or other matter implicated in evacuation channel blockage.

In some embodiments of the invention, one or more irrigating channels transport irrigating material to a distal end of a colon cleaning system. One or more evacuation channels return the material with colon waste. In some embodiments, transport is reversible and/or otherwise variably controllable. Potentially, control of evacuation flow clears or prevents blockages during use with reduced need to interrupt a procedure due to equipment failure.

An aspect of some embodiments of the invention relates to sensors of conditions in a colon cleaning system and/or its operating environment.

In some embodiments of the invention, one or more sensors detect pressure within the bowel or cleansing system. Additionally and/or alternatively, sensors detect another property of material inside or outside the system; for example: flow, flow rate, temperature, conductivity, optical density, spectral properties, pH, and/or osmotic pressure. In some embodiments, sensors monitor system components. Examples include a pump volume meter, a fill level sensor, and/or a valve or intake position sensor.

In some embodiments, distal sensors connect to a workstation by wire or wireless radio. Additionally and/or alternatively, sensors in a workstation sense pressure through one or more sensing ducts with distal outlets.

It is a potential advantage to provide sensors capable of indicating potential and/or recently formed blockages to flow at an early stage of blockage formation. Optionally, blockage comprises blocking material carried by evacuated fluid. Optionally, blocking material comprises a clot. Optionally, blocking material comprises a build-up on an evacuation lumen wall. Potentially, early detection allows initiating action to purge a forming blockage before it becomes an impediment to evacuation function. Potentially, blocking material is more easily purged, the earlier its presence is sensed. Optionally, blockage comprises a restriction in flow due to a change in the shape of an evacuation lumen. The change may be, for example, due to kinking or reduced pressure in an evacuation lumen. Potentially, a change in the pressure exerted in an evacuation lumen reduces the flow restriction. Optionally, a restriction in flow comprises drag due to turbulence in flow. Potentially, a change in the pressure exerted in an evacuation lumen reduces turbulence and associated drag.

An aspect of some embodiments of the invention relates to use of sensed and/or determined system statuses for determining control signals that operate components of a colon cleaning system. Sensing changes during controlled operation optionally allows directed and/or closed-loop control of the system.

In some embodiments of the invention, sensor data are processed to determine a status. Exemplary statuses include channel blockage and bowel pressure. In some embodiments, the determination of blockage in an evacuation channel of the system elicits signaling to operate colon cleaning system components for clearing the block. For example, a determination of a tip block elicits signaling to terminate suction. Optionally, control signals are modulated based on specifics of a status determination. Determined specifics include, for example: a blockage being full, partial or developing; blockage location; and/or blockage type, for example waste particle or captured tissue.

It is a potential advantage to control actions for purging blockages and potential blockages for reduced interference with evacuation flow. A build-up of material in the colon can slow procedure progress and/or interfere with viewing while purging is completed. Material buildup leading to increased pressure is potentially a danger to the patient.

In some embodiments of the invention, sensing and control have feedback interactions. In one example of feedback, flow direction is cycled in a channel until a determined blockage condition is relieved. In another example, controlled probing pressure changes are commanded. The rate at which they are sensed to propagate through a channel potentially indicates relative volumes of incompressible fluid and compressible gas, and/or flow capacitance. Potentially, this can indicate foaming of evacuating fluid. Parameters of timing, pressure and/or flow control are optionally varied according to this or another actively sensed status. In another example, control signals balance the relative efficacy sensed for two or more subsystems. Optionally, this is useful to balance irrigation volume with evacuation volume.

In some embodiments, a fault status-examples include bowel overpressure, an unremovable blockage, or unexpected sensor values-leads to a control response. Fault responses include, for example: shutting down, pausing, reducing, and/or increasing activity of a system component.

In some embodiments, feedback from sensors is used to control flow in a fluid delivery system which delivers fluid that mixes with fecal matter for evacuation. In some embodiments, the delivered fluid comprises gas, for example, carbon dioxide and/or air. In some embodiments, the delivered fluid comprises a liquid, for example water or saline. In some embodiments of the invention, the supply comprises a mix of gas and water, for example, water and gas jetted together through a fluid supply conduit. In some embodiments, sensing of pressure inside the intestine is used to detect an actual, immanent, developing and/or potentially developing over-pressurization of the intestine (any of which is referred to herein as an overpressure condition). An overpressure condition comprises, for example, a pressure above a safety limit, and/or a changing pressure which appears likely, based on a rate of change, to soon reach a safety limit. In some embodiments, a system reacts to an overpressure condition by reducing a rate of fluid supply, by changing a ratio of gas to liquid in a fluid supply (for example, reducing an amount of gas), and/or by terminating the supply of fluid. In some embodiments, a ratio of evacuation rate to fluid supply is adjusted. Optionally, the fluid supply rate remains fixed while an evacuation rate is increased. In some embodiments of the invention, fluid supply is halted. In some embodiments of the invention, another limit is imposed (additionally or alternatively): for example, a limit to the length of an irrigation sequence. In some embodiments of the invention, a sensor is placed at the distal tip of the cleaning system (where it can be positioned, for example, at the end of an intestine), and outside of the cleaning system lumens, where it is directly exposed to the pressure within the bowel. The sensed pressure is used as a basis for the detection and feedback control of overpressure. In some embodiments, overpressure is controlled by appropriate actions (increase and decrease of material flow) commanded by a controller to balance the input and output of the cleaning device's pressure and/or fluid sources to the colon, while evacuation and irrigation are taking place. In some embodiments, control of irrigation comprises stoppage or reduction of irrigation while purging actions to clear and/or prevent an evacuation lumen blockage take place.

In some embodiments, operator input affects control signals; for example: an onset, duration and/or intensity of irrigation and/or suction. In some embodiments, parameters of device operation include control by a timer, for example, within a preprogrammed cleaning cycle, or as a limiting safety mechanism to protect the bowel from overfilling.

An aspect of some embodiments of the invention relates to the arrangement of the lumenal cross-section area through which evacuation and/or irrigation occur.

In some embodiments of the invention, a plurality of evacuation channels replace a single larger channel. Optionally, a lower maximal probe diameter is allowable thereby for the same overall cross-section. Diameter is a factor in probe flexibility, which in turn may affect colonoscopy procedure completion rates. Potentially, a plurality of evacuation channels allows optional independent operation and/or increased reliability of operation.

Before explaining at least one embodiment of the invention in detail, it is to be understood that the invention is not necessarily limited in its application to the details of construction and the arrangement of the components and/or methods set forth in the following description and/or illustrated in the drawings. The invention is capable of other embodiments or of being practiced or carried out in various ways.

For convenience of exposition, cleaning systems and methods described herein are sometimes referred to as "colon cleaning" systems and methods. Colon cleaning is contemplated as a common use of embodiments of the invention. However, it is to be understood that methods and devices taught herein may also be used to clean other portions of an intestine and/or other body lumens. Accordingly, the term "colon cleaning" as applied to these methods and devices encompasses cleaning not only the colon, but also other portions of an intestine and/or other body lumens.

### Reference Embodiment of a Colonoscope Cleaning System

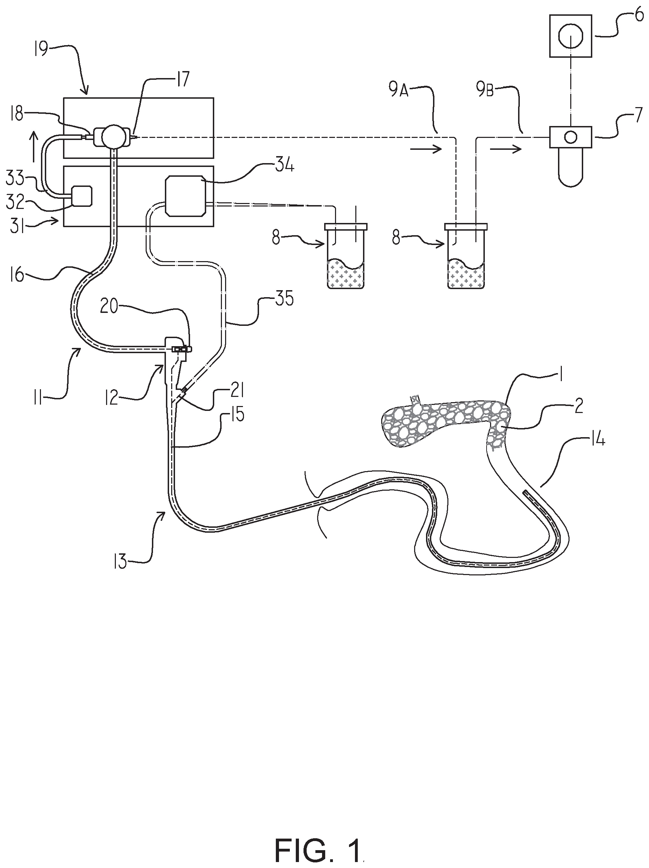

Reference is now made to *Figure 1*, which schematically illustrates a colon cleaning system comprising colonoscope work station **19** and a cleansing work station **31**, according to some exemplary embodiments of the invention.

In some exemplary embodiments of the invention, the colonoscope and cleansing work stations **19**, **31** connect via water or gas pipe **33**. Optionally, pipe **33** connects at one end to the water or gas pump **32** of cleansing work station **31**, and at the other end to colonoscope **19** water or gas inlet **18**. Optionally, colonoscope umbilical cord **11** comprises internal tubes **16**. Colonoscope insertion tube **13** connects to colonoscope umbilical cord **11** via colonoscope handle **12**. In some embodiments, handle **12** comprises vacuum valve **20** and work channel inlet **21**. Colonoscope tip **14** is depicted navigating through a colon **1** full of fecal matter **2**.

In some exemplary embodiments, cleansing work station pump **34** connects via pipe **35** to colonoscope work channel **21** at one end, and to fluids container **8** at the other end. In some embodiments, colonoscope vacuum inlet **17** connects via fluid line **9A** to fluid container **8**. Optionally, suction wall pump **6** connects through a regulator **7** via lines **9B** to container **8**.

In some embodiments of the invention, operating pump **32** augments irrigation of the colon with a supply of gas and/or water through pipe **33** and inlet **18** to colonoscope work station **19**. In some embodiments, irrigation material is supplied via internal pipes **16** and working channel **15** all the way to colonoscope tip **14**, augmenting cleaning colon **1** of fecal matter **2**. In some embodiments of the invention, supplied gas comprises, for example air or carbon dioxide.

In some embodiments of the invention, fecal matter is evacuated through colonoscope working channel **15** and internal pipes **16** through vacuum inlet **17**. Optionally, inlet **6** provides lowered pressure via regulator **7** through pipe line **9B** and **9A** to suck fecal matter into fluids container **8**.

In some embodiments, evacuation is augmented as needed by cleansing working station **31**. Optionally, operating pump **34** and/or opening inlet **21** creates pressure differentials which move material through pipe **35** into fluids container **8**.

According to the embodiment of the invention, colon cleaning systems comprise colonoscopes or are parts of colonoscopes. Alternatively and/or additionally, some embodiments presented herein may be implemented as cleaning systems independent of and optionally usable with colonoscopes.

In some embodiments of the invention, some components are provided by a colonoscope with which a colon cleaning system is used. For example, a cleaning system according to some embodiments of the invention uses a working channel of a colonoscope as an irrigation channel and/or as a matter evacuation channel (also described herein as an evacuation lumen). In some embodiments, components are provided separately from the colonoscope. For example, a cleaning system according to some embodiments of the invention uses channels or other tools external to a colonoscope as irrigation channels, evacuation channels and/or other system components.

### Sensors and Motive Devices for Exemplary Cleaning Systems

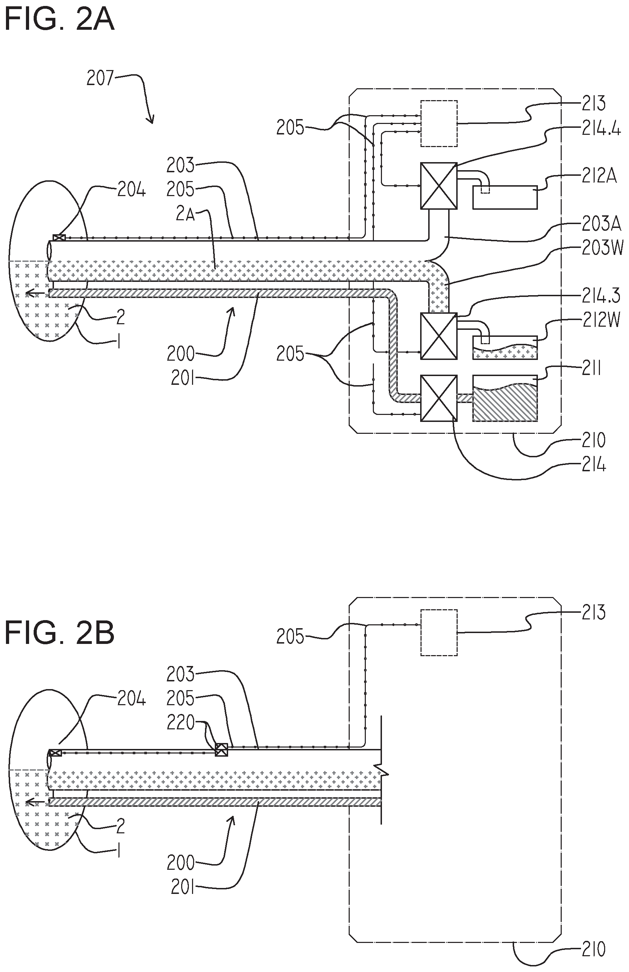

Reference is now made to *Figure 2A*, which schematically illustrates a colon cleaning system **207** with sensors in accordance with some exemplary embodiments of the present invention.

In some embodiments of the invention, cleaning system **207** comprises one or more motive devices for moving fluid through channels (for example, pumps **214**, **214.3**, **214.4**). Optionally, motive devices are operated from signals sent by a controller **213**. Optionally, controller **213** determines controlling signals based on inputs from one or more sensors **204**.

In some embodiments of the invention, cleaning system **207** comprises an insertable portion **200** insertable to colon **1** of a patient, and external work station **210**. In some embodiments, system **207** comprises an irrigation pipe **201** through which an irrigation fluid such as water and/or other liquid may be provided into colon **1**. Irrigation pipe **201** is connectable to a source of water or other liquid **211**.

### Exemplary Motive Devices

System **207** optionally comprises one or more waste collection devices **212A**, **212W** for collection of waste matter (including fecal matter) evacuated from colon **1**. System **207** optionally comprises an evacuation pump **214.3**, **214.4** which provides suction for evacuating matter from colon **1** through system **207**. During some phases of a colon cleaning operation, pump **214.3**, **214.4** evacuates fecal matter (optionally loosened and/or transported by liquid from irrigation pipe **201**) from colon **1** into waste collection device **212A**, **212W** via an evacuation channel **203**.

In some embodiments of the invention, evacuation pump **214.3**, **214.4** is external to the device. Optionally, evacuation pump **214.3**, **214.4** is a connection to a vacuum supplied by a distant pump, such as is available from a vacuum source connector available in many hospitals and clinics. In this case, references herein to controlling pump **214.3**, **214.4** (e.g. starting and stopping its suction-creating operation) may be understood to refer to controlling a valve connecting an evacuation tube of a cleaning system to such a distant vacuum source.

In some embodiments of the invention, system **207** comprises a pressure sensor **204**, positioned on or near a distal end of evacuation channel **203**, for measuring pressure in colon **1**. In some embodiments, sensor **204** reports pressure readings to a controller **213**. Optionally, controller **213** controls operation of one or more of pumps **214**, **214.3** and/or **214.4**. Control of operation comprises, for example, starting, stopping, and/or modifying the speed or power of a pump **214**, **214.3**, **214.4**. Optionally, control of operation is based on pressure readings detected at sensor **204**.

In some embodiments, controller **213** comprises a processor for calculating and sending commands to pumps **214**, **214.3**, **214.4** based on and/or as a function of sensor readings from sensor **204**. Optionally, control is based on other sensing mechanisms and/or inputs in system **207**, for example commands input by a user.

In some embodiments, system **207** comprises a purge pump **214** for inputting water and/or other fluid to system **207**. Optionally, system **207** comprises a fluid source **211** of, for example: water, another liquid, a mixture of liquid and gas, and/or gas such as pressurized air or carbon dioxide. Optionally, purge pump **214** introduces fluid under pressure to purge portions of system **207** such as evacuation channel **203**. Purging is activated, for example, when a blockage of evacuation channel **203** is determined by controller **213** based on input from sensor **204** or another source. According to the embodiment, controller **213** controls pumps **214**, **214.3**, **214.4** via wired or wireless connections **205**.

In some embodiments of system **207**, some or all of controller **213**, purge pump **214**, evacuation pump **214.3**, **214.4**, and waste collection device **212W** are grouped together in a work station **210**.

### Exemplary Sensor Locations

In *Figure 2A*, exemplary sensor **204** is shown positioned at a distal end of evacuation channel **203** and external to that channel. Sensor **204** in this position directly measures pressure within colon **1** when channel **203** is inserted in colon **1**. In some embodiments of the invention, sensor **204** is an electronic pressure sensor.

Reference is now made to *Figures 2B-2D*, which schematically illustrate additional or alternative sensor placement positions within a cleaning system **207** such as that of *Figure 2A*, according to some exemplary embodiments of the invention.

In *Figure 2B*, exemplary sensor **204** is positioned at or near a distal end of channel **203** and inside channel **203**. In this configuration, sensor **204** measures and reports pressure within a distal portion of channel **203**. Optionally, sensor **204** is connected by a wired connection **205** through a connector **220** to controller **213**. In some embodiments of the invention, a wired connection **205** runs inside channel **203** along all or part of its length. The connection transfers outside of channel **203** (for example, through a connector **220**) at a point proximal to the portion of channel **203** inserted into a colon during use. In some embodiments of the invention, connector **220** is an electrically conductive connection. In some embodiments of the invention, the connection is wireless; for example, a radio or optically coupled connection.

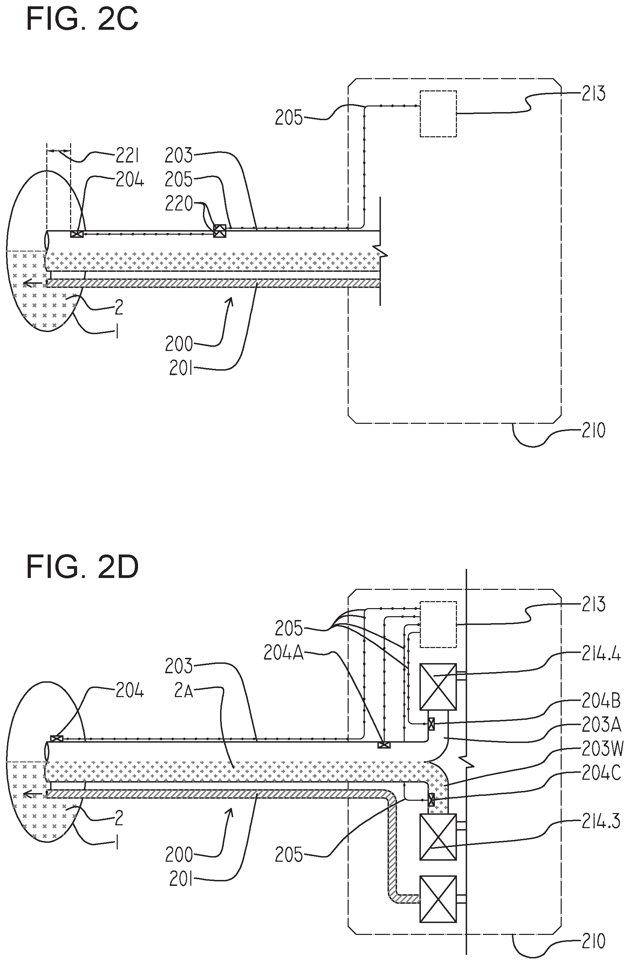

In *Figure 2C*, exemplary sensor **204** is positioned within evacuation channel **203** and at a distance **221** from a distal end of channel **203**. According to the embodiment, distance **221** is, for example, 0 mm, 1 mm, 2 mm, 5 mm, 10 mm, 20 mm, 30 mm, any distance in between, or a larger or smaller distance.

In *Figure 2D*, exemplary sensors **204A**, **204B**, **204C** are positioned at or near a proximal end of channel **203**. Three sensors are shown: one **204A** at a proximal end of channel **203**, one **204B** in a sub-channel connecting evacuation pump **214.4** to channel **203**, and one **204C** in a sub-channel connecting purge pump **214.3** to evacuation channel **203**. Optionally, sensor **204** is placed outside channel **203**. Potentially, this allows sensing of pressure directly within the bowel. This is a potential advantage, for example, to allow pressure to be sensed such that over-pressurization of the bowel can be prevented. Prevention of over-pressurization is, for example, by termination and/or reduction of the delivery of gas or fluid to the bowel (for example, by irrigation pipe **201**). In some embodiments, delivery by irrigation pipe **201** (which is optionally a jetting delivery system), or another gas or fluid delivery system, such as an insufflation tube to maintain bowel inflation, is reduced and/or terminated when pressure is determined to be at, near, and/or nearing (rising to approach) an over-pressure condition. In some embodiments, pressure rising to approach over-pressure is determined on the basis of a difference of pressure measured during at least two different times. Additionally or alternatively, evacuation rate is increased at or near a sensed over-pressure condition. Potentially this prevents over-inflation, while allowing cleaning to continue uninterrupted.

In some embodiments of the invention, sensor placements shown in *Figures 2A-**2D* and/or other figures herein are combined. In some embodiments of the invention, for example, a single sensor **204** is positioned at a position as shown in one of *Figures 2A-**2D*. In some embodiments, a plurality of sensors are positioned at a position shown, for example for redundancy. Additionally and/or alternatively, sensors may be positioned at a plurality of positions as shown.

### Exemplary Sensor Devices

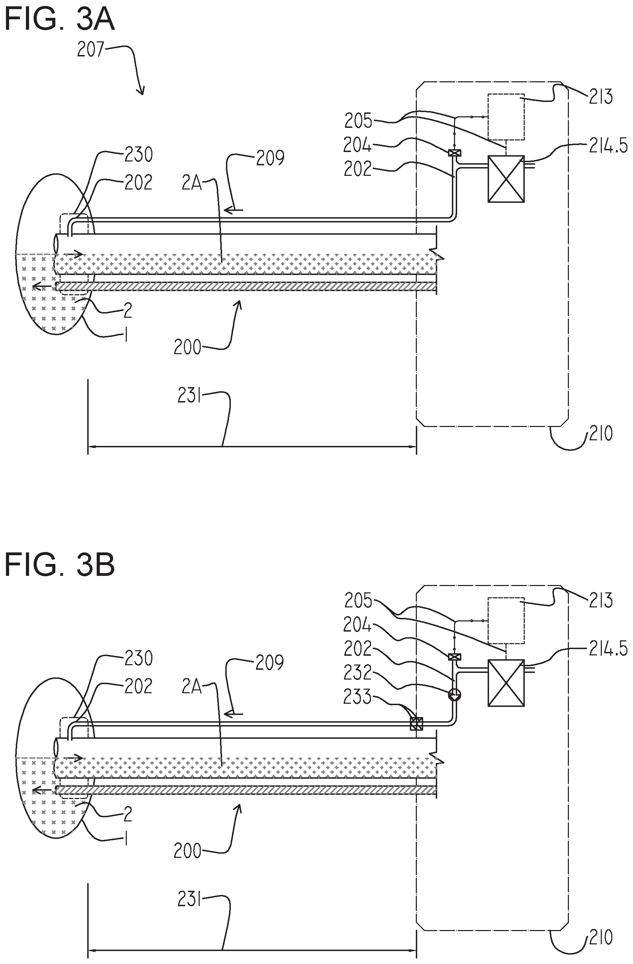

Reference is now made to *Figures 3A-3B*, which schematically illustrate a colon cleaning system **207** including a remote pressure-sensing sensing module, in accordance with some exemplary embodiments of the present invention.

In some embodiments of the invention, pump **214.5** is actuatable to generate positive pressure in tube **202**, extending from pump **214.5** to a distal end of evacuation channel **203**. Optionally, pump **214.5** is positioned within work station **210**. Optionally, tube **202** is connected to evacuation channel **203** via a head device **230**. The working fluid pumped by pump **214.5** is, for example, air, carbon dioxide, water, or another fluid.

Positive pressure potentially produces flow **209** in tube **202**. During colon cleaning, suction provided by an evacuation pump (not shown) causes flow **209** to enter the distal end of evacuation channel **203**. From there, it is drawn the length of channel **203** and out of system **207**.

While flow **209** continues uninterrupted, pressure buildup in tube **202** and channel **203** is avoided. A flow or pressure sensor **204** senses pressure or rate of flow **209** and reports to controller **213**.

In some embodiments of the invention, when a clog or obstruction blocks or partially blocks evacuation channel **203**, flow is not exhausted or is inefficiently exhausted from channel **203**. Pressure builds and/or flow slows in channel **203** and/or tube **202**. Pressure/flow sensor **204** detects a change.

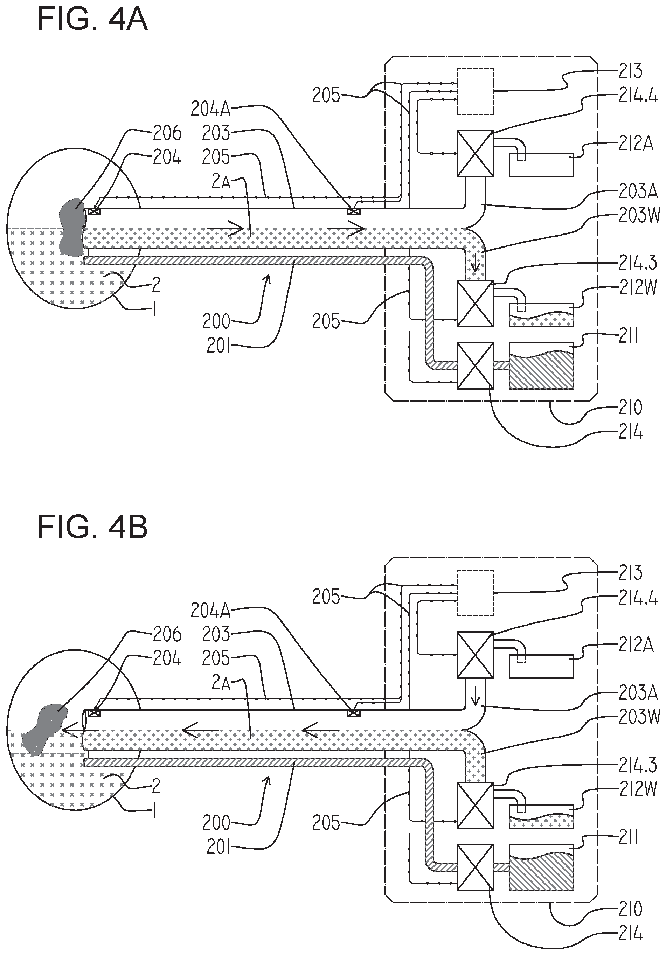

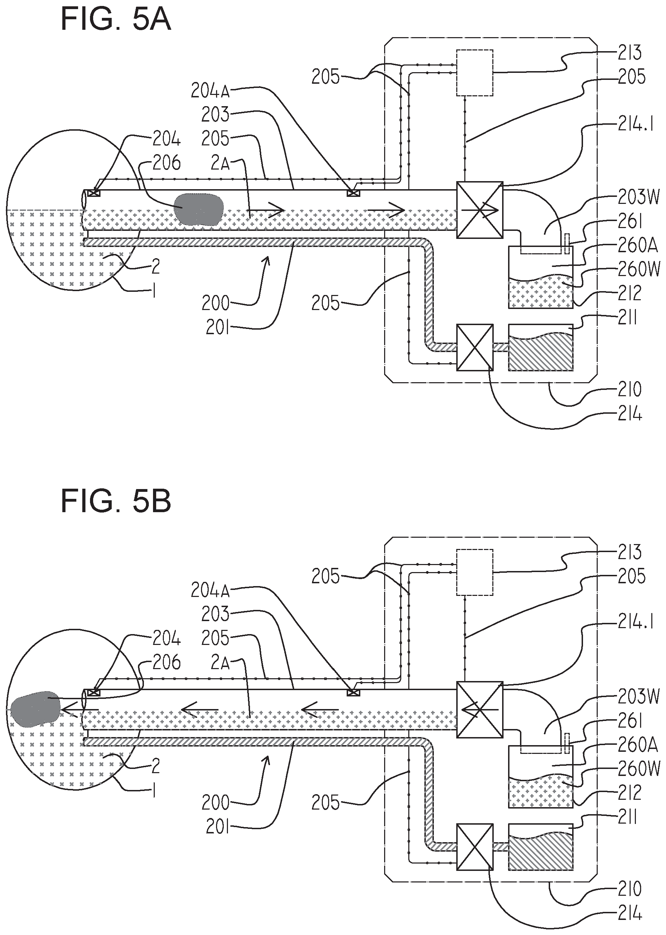

In some embodiments, the sensor **204** reports a change and/or a changing value to controller **213**. In some embodiments, controller **213** determines the existence of a blockage from a detected drop in flow or increase in pressure. Optionally, controller **213** initiates corrective action to eliminate the blockage and restore flow **209**, as detailed in relation to, for example, *Figures 4A-4F* and *5A-5C*.

A potential advantage of a configuration using remote sensing is that sensors and associated electronics occupy portions of system **207** outside the body. Potentially, this reduces regulatory concerns over the introduction of electronic devices to a body cavity. The length of tube **202** may be, for example, more than 2 m, 3 m, 4 m, or any length in between.

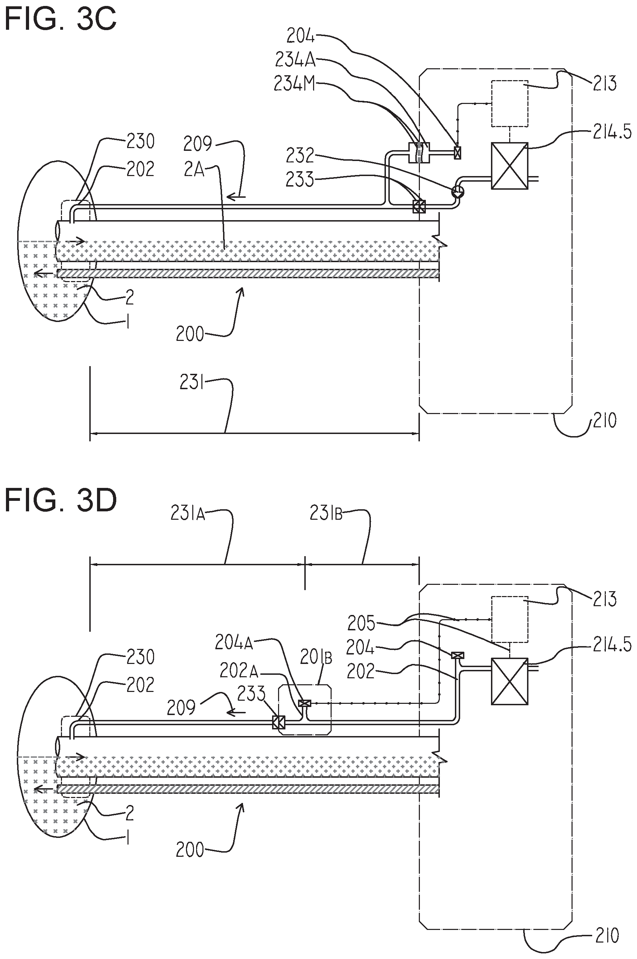

Reference is now made to *Figure 3C*, which schematically illustrates a variation of a sensing module for colon cleaning system **207**, according to some exemplary embodiments of the invention.

It is a potential advantage to prevent fault conditions from contaminating the interior of the cleaning system.

In some embodiments of the invention, sensor **204** is protected from potential backflow into tube **202** by a protective membrane **234M** which comprises a barrier to back-flowing material. In some embodiments, membrane **234M** is sufficiently flexible that pressure changes are communicated across it to chamber **234A** where sensor **204** is located.

In some embodiments of the invention, a check valve **232** resists backflow through coupler **233**. If back pressure overcomes the forward pressure of pump **214.5**, the valve closes, preventing flow from passing across connector **233** and contaminating the system.

Reference is now made to *Figure 3D*, which schematically illustrates a variation of a sensing module for colon cleaning system **207**, according to some exemplary embodiments of the invention.

In some embodiments of the invention, flow provided by pump **214.5** and flowing through tube **202** is measured by sensor (pressure and/or flow meter) **204A**, distal to sensor **204**. Optionally, sensor (pressure and/or flow meter) **204A** is mounted in an independent work station **201B**. From there, it connects through a connector **233** and an additional flow tube **202A** to an outlet in a distal portion of evacuation channel **203**.

A potential advantage of this embodiment is reduction of the distance **231A** between the sensor **204A** and the distal outlet of tube **202** in channel **203**. The reduced distance is shorter than the sum of the distances **231A** and **231B**. Potentially, therefore, pressure and/or flow readings from sensor **204A** will respond more accurately to blockages in channel **203**, producing more accurate sensor readings.

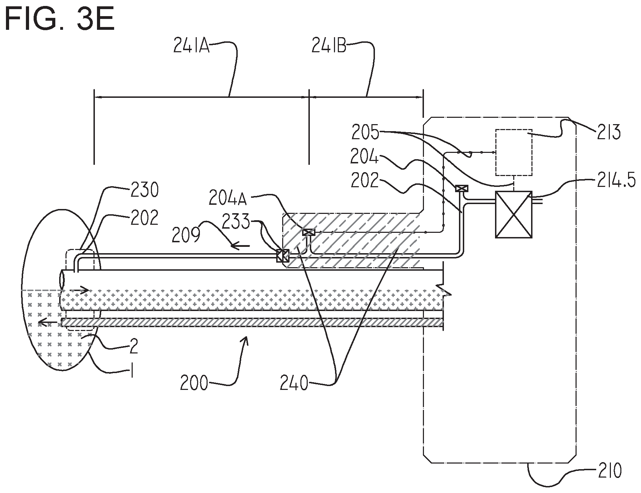

Reference is now made to *Figure 3E*, which schematically illustrates a variation of a sensing module for colon cleaning system **207**, according to some exemplary embodiments of the invention.

In some embodiments of the invention, flow provided by pump **214.5** and flowing through tube **202** is measured by sensor (and/or flow meter) **204A**, distal to sensor **204**. Optionally, sensor (and/or flow meter) **204A** is mounted in an extension **240** of work station **210**. Sensor **204A** connects through a connector **233** to an outlet in a distal portion of evacuation channel **203**.

A potential advantage of this embodiment is shortened distance **241A** between the sensor **204A** and the distal outlet of tube **202** in channel **203**. The distance is shortened relative to the sum of the distances **241A** and **241B**. Potentially, therefore, pressure and/or flow readings from sensor **204A** will respond more accurately to blockages in channel **203**, producing more accurate sensor readings.

*Figures 2A-3E* illustrate placements of sensors, described hereinabove in connection to exemplary sensor types and configurations, including pressure sensing and flow sensing. In some embodiments of the invention, sensors of one or more alternative types detect another parameter related to material inside or outside the system. Examples include: flow rate, particle sensing, temperature, conductivity, optical density, spectral properties, pH, and/or osmotic pressure. In some embodiments of the invention, a probe energy source appropriate to the sensor type, for example a source of illumination for an optical probe, is appropriately disposed.

According to the embodiment, sensor types relate to the operation of a colon cleansing system in the following exemplary aspects:
- A flow rate sensor determines flow by velocity rather than volume. A low flow rate potentially indicates a blockage, for example, in the lumen of an evacuation channel.
- A particle sensor provides information on particles within a sensing region. According to the embodiment, a particle sensor is used, for example: to provide a rate of particle motion, potentially indicating a blockage; to provide particle size estimation, potentially indicating a need for more energetic breaking of particles and/or an increased risk of blockage; and/or to provide a count or estimate of particle density, potentially indicating an increased risk of blockage.
- A temperature sensor provides a temperature within a sensing region. Sensed changes in fluid temperature potentially indicate, for example, a longer or shorter dwell time of fluid in the body. Optionally, dwell time in the body is indicated by the amount to which an irrigation fluid temperature, beginning at a temperature different from body temperature, has equilibrated to human body temperature. Potentially, information on fluid dwell time assists a determination of net flow in the system.
- A conductivity sensor provides an indication of electrical conductance, for example the conductance of evacuating waste fluids. A change in conductance potentially indicates, for example: a change in the relative mixture of gas and fluid in evacuated discharge (including indicating foaming and/or the evacuation of substantially all fluid) and/or a change in the balance of irrigating fluid and bowel fluid in the evacuation channel.
- A pH sensor provides an indication of ionic concentrations in a fluid. A change in pH potentially indicates, for example, a change in the balance of irrigating fluid and bowel fluid in the evacuation channel.
- An osmotic sensor provides an indication of solute concentration in a fluid. A change in solute concentration potentially indicates, for example, a change in the balance of irrigating fluid and bowel fluid in the evacuation channel.
- An optical density sensor provides an indication of the optical density of a fluid. Measurement of optical density (and in particular turbidity) potentially provides, for example, an indication of the waste content of evacuated waste fluid, and/or foaming in the fluid. A range of optical densities is potentially an indication of a greater potential for a blockage to develop. In particular, such a range may be set at the low end by a relative lack of particles in a channel, and at a high end by turbidity sufficient to indicate that particles are small and unlikely to form blockages. Additionally or alternatively, a high optical density is potentially an indication of foaming.
- A spectral sensor indicates light spectral properties, for example, optical density as a function of light wavelength. Measurement of spectral properties potentially reveals the concentrations of one or more substances in evacuated irrigation matter. Optionally, illumination, sensing, and processing are tuned to the properties of hemoglobin. Potentially, sensed hemoglobin in evacuated irrigation material provides an indication of hemorrhaging, a serious complication in a small proportion of colonoscopy procedures. In some embodiments, a foaming fluid, dominated by scatter having spectral properties similar to the illumination light, is distinguishable from a particle-laden fluid, having spectral properties which absorb light according to the particle content.
- Sensing of pump operation and/or pumping efficacy parameters (for example, of a rate of rotor rotation, or of acceleration) potentially provides an indication, for example, of the density of material the pump is moving. Potentially, reduced density of the material indicates foaming.

### Exemplary Blockage Removal

Reference is now made to *Figures 4A-4D*, which schematically illustrate removal of blockages from an evacuation channel **203** of a colon cleaning system, according to some exemplary embodiments of the invention.

During cleansing, pump **214** supplies irrigation liquid from fluid container **211** via tube **201** to the colon **1**, while pump **214.3** evacuates fecal matter **2** combined with irrigating fluid **2A** via tube **203** into collecting container **212W**.

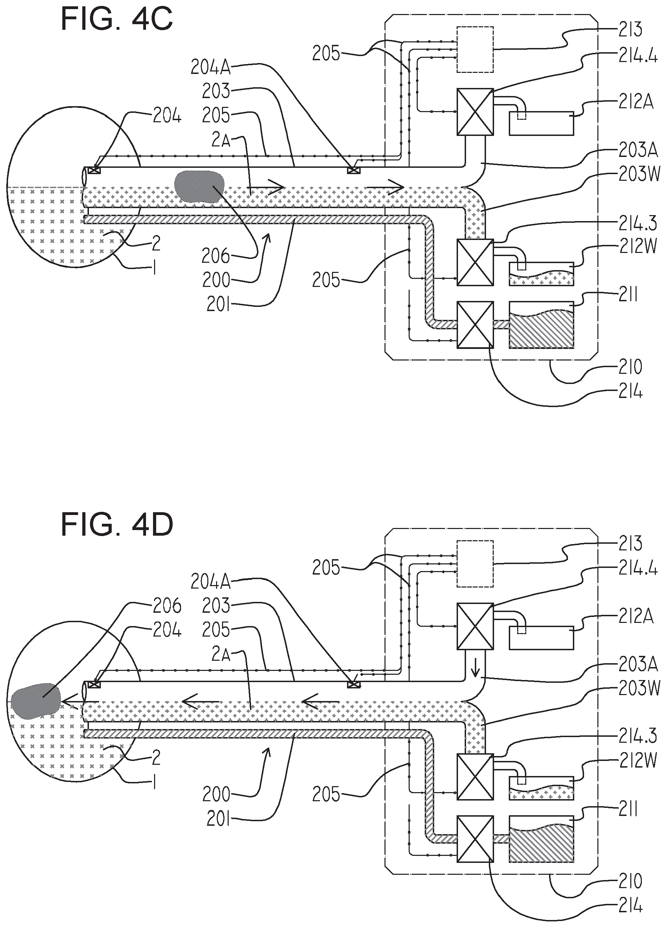

In *Figure 4A*, evacuation channel **203** is blocked or partially blocked by fecal matter **206** covering an intake aperture. In *Figure 4C*, evacuation channel **203** is blocked or partially blocked by fecal matter **206** lodged and/or wedged inside.

When there is blockage of tube **203**, pressure sensors **204** and/or **204A** read changes in pressure level, and signal those changes to controller **213**, for example via wire **205**. Based on the sensed data, controller **213** potentially makes a determination that a blockage exists.

Having determined that a blockage exists, controller **213** stops pump **214**, **214.3** operation and starts operation of pump **214.4**. Pump **214.4** supplies liquid from container **212A** into evacuation tube **203** under reversed pressure, potentially pushing a developed or developing blockage **206** away (*Figures 4B* and *4D*).

It should be noted that as evacuation channel **203** is inserted into an intestine, it potentially acquires constrictions and/or partial collapses. Narrowed portions potentially result, for example, from twists of channel **203** imposed by colon anatomy and/or colon position, and/or from body organs or other objects pressing on channel **203**. These narrowed portions tend to catch and hold large pieces of passing fecal matter at the point where they are too large to pass. Potentially, therefore, fecal matter **206** is primarily blocked in one direction of travel, and susceptible to backward expulsion from regions which it has reached and cannot pass. The blockage is particularly susceptible to backward expulsion if corrective action is taken before there is a chance for it to become impacted from behind by following particles.

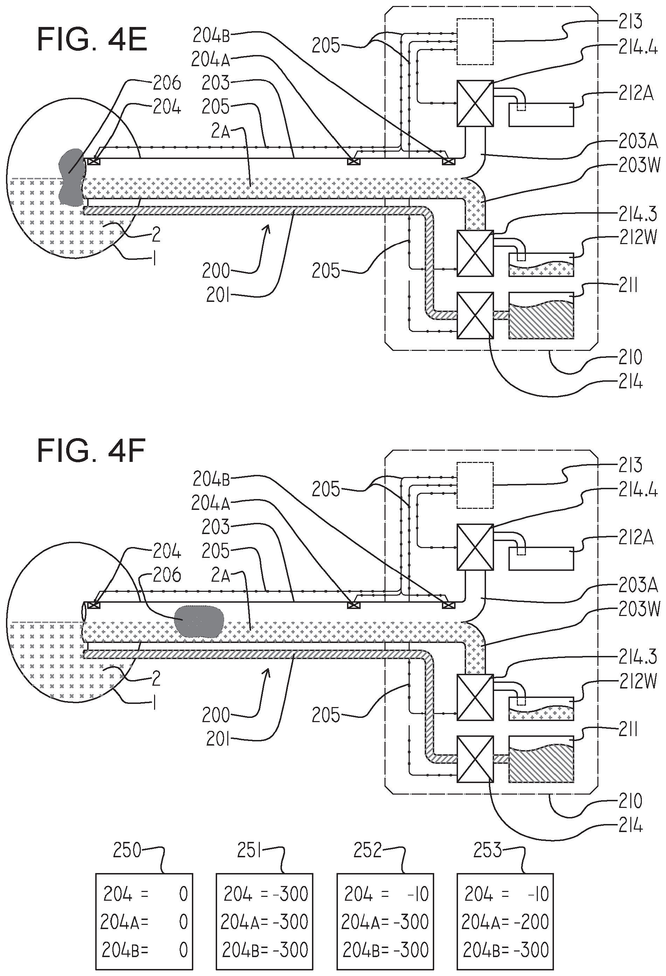

Reference is now made to *Figures 4E-4F*, which schematically illustrate details of how clot material positioning in or on channel **203** affects readings of exemplary system sensors **204**, **204A**, **204B**, according to some exemplary embodiments of the invention. Also given are sets of exemplary sensor readings **250**, **251**, **252**, **253**, used in determination of status by controller **213**.

The exemplary system sensors comprise pressure sensors in distal, proximal, and medial portions of a channel **203**-sensors **204**, **204B**, and **204A**, respectively. Sensor types are exemplary, and could be different according to the embodiment.

Sensor reading set **250** reflects the status of a system which is not flushing, evacuating, or purging. Flowmeter **204A** shows zero flow, and pressure sensors **204** and **204A** and **204B** report zero difference from ambient pressure. A flow meter, if present (for example at the location of **204A**), would indicate no flow.

Sensor reading set **253** comprises exemplary readings reflecting the status of a system where normal evacuation flow is occurring during colon cleaning. Sensors **204**, **204A**, and **204B** read -10 mbar, -200 mbar and -300 mbar respectively relative to ambient pressure. A flow meter, if present, would indicate flow.

Two exemplary abnormal situations are also shown.

Sensor reading set **251** comprises exemplary sensor readings reflecting the status of a system in which fecal matter **206** (*Figure 4E*) clogs the tip of evacuation channel **203**. Sensors **204**, **204A**, **204B** all read a low pressure of -300 mbar relative to ambient pressure. A flow meter, if present, would indicate no flow.

Sensor reading set **252** comprises exemplary sensor readings reflecting the status of a system in which fecal matter **206** (*Figure 4F*) clogs the interior of evacuation channel **203**. Sensors **204**, **204A**, and **204B** read -10 mbar, -300 mbar and -300 mbar respectively relative to ambient pressure. A flow meter, if present, would indicate no flow.

Blockage potentially begins as a partial occlusion, developing over time into greater occlusion and/or increased resistance to purging. In some embodiments of the invention, sensing is performed at a sufficient frequency to detect intermediate pressure values between a blocked and an unblocked state. The sampling rate is, for example, 5 Hz, 10 Hz, 20 Hz, 50 Hz, 100 Hz, 200 Hz, a frequency in between, or a higher or lower frequency. In some embodiments of the invention, the detection of intermediate pressure values allows determination that a blockage is in development. The determination is potentially before the blockage begins to significantly impair the flow of evacuation fluid. In some embodiments of the invention, the determination is before a blockage reaches a particular threshold of flow impairment, for example 10%, 20%, 40%, 50%, 80%, or another higher or lower threshold of flow impairment. In some embodiments of the invention, the determination is within a particular time interval after reaching a particular threshold of flow impairment, for example: within 10-20 msec after reaching at least 90-95% occlusion, within 15-40 msec after reaching at least 85-95% occlusion, within 25-50 msec after reaching at least 70-80% occlusion, within 50-100 msec after reaching at least 50% occlusion, or within 50-100 msec after reaching at least 90% occlusion. A potential advantage of early detection of a developing blockage is that action can be taken to purge the evacuation lumen before the blockage becomes securely wedged in place. Potentially, a small obstruction (for example, of 15% of the lumen diameter), begins a chain-reaction leading to a full blockage: for example, a 90%-of-lumen-diameter particle, which previously passed freely, is potentially impeded upon encountering a 20%-of-lumen-diameter blockage, becoming itself an obstruction. At this stage, even if occlusion already approaches 100%, the blockage is potentially reversed more easily if detection is rapid, as additional particles coming from behind may add to the developing block, increasing its resistance to purging.

Another potential advantage of early detection of a developing blockage is that the action required to dislodge a small block can be less aggressive, so that interference with cleaning and evacuation is less interfered with.

Reference is now made to *Figures 5A-5C*, which schematically illustrate a single pump **214.1** operable as an evacuation and purging pump, in accordance with exemplary embodiments of the invention.

In some exemplary embodiments of the invention, pump **214.1** has bi-directional pumping capability to pump material in both distal and proximal directions, according to its mode of operation.

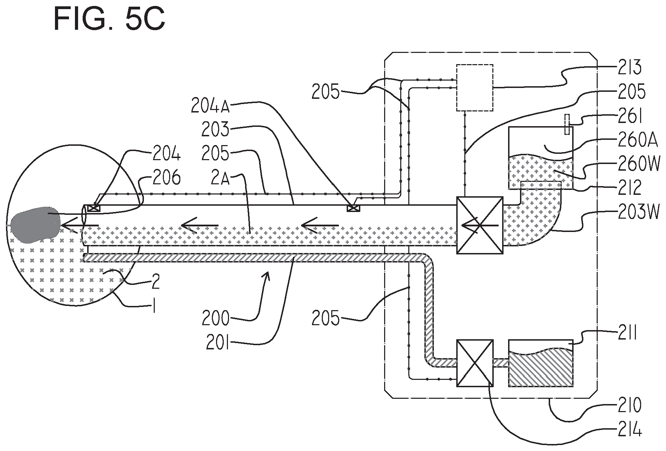

In some exemplary embodiments, pump **214.1** pulls waste proximally to evacuate it during an irrigation phase of colon cleaning. In some embodiments, upon a determination to purge evacuation channel **203**, controller **213** reverses the pumping direction of pump **214.1**, producing distal-acting pressure, and possibly flow. The determination is, for example, in response to a determined blockage **206** as in *Figure 5A* and/or a user's manual command. The distal-acting pressure and/or flow pushes against a determined blockage **206**, potentially dislodging it from within channel **203** (*Figures 5B* or *5C*).

Optionally, the purging pulse is brief, for example, 50 msec, 100 msec, 200 msec, 500 msec, 1000 msec, 2000 msec, any time in between, or a larger or smaller time. It is a potential advantage to keep purging no longer than necessary to dislodge a blockage, to avoid pushing large quantities of material back into the colon. Optionally, alternating pulses of suction and purging are delivered. The total pulse number is, for example: 2 pulses, 4 pulses, 8 pulses, 20 pulses, 40 pulses, 80 pulses, any number in between, or a larger or smaller number of pulses. It is potential advantage to deliver a sequence of pulses, to agitate and/or batter blocking matter. Potentially, a sequence of pulses breaks apart clumps, unblocking the channel.

In some embodiments, the purging material container **212** contains both gas **260A** and fluid **260W**. In some embodiments, tube **203W** is positionable to selectively pull in either gas **260A** or fluid **260W** during a purging operation. For example, tube **203W** in *Figure 5B* is pushed down into fluid **260W** to extract fluid, or raised into gas (for example air) **260A** to extract gas. Optionally, container **212** is invertible, for example, held in the hands. Holding container **212** as in *Figure 5B* allows, for example, gas **260A** to be extracted for purging. Inverting container **212** as in *Figure 5C* allows fluid **260W** to be extracted for purging.

In some embodiments, a vent **261** on container **212** allows accumulated gas to be vented, and/or to be drawn into container **212** as needed during operation.

In some embodiments, a gas input is provided to channel **203**. Potentially, a liquid:gas mixture is provided in a ratio of, for example, 10:1, 4:1, 2:1, 1:1, 1:2, any ratio in between, or another higher or lower ratio. It is a potential advantage to mix gas and fluid for more effectively breaking apart clumps of tissue. Applicant's experiments have shown that such a gas/liquid mixture can be effective in dislodging and/or breaking up clumped fecal material.

### Other Status Determinations and Responses

The foregoing examples illustrate how a controller can make a status determination based on the combination of data from a group of sensors, and/or from a single sensor. In the case where a group of pressure sensors is used, the existence and general location of a blockage can be determined. In the case of a single flow sensor, the existence of a blockage can be determined. Command signals are issued by controller **213** according to the determined status.

In some embodiments of the invention, the determination of a status leads to one of several, optionally graded response from the controller **213**. The response depends on the particulars of the sensed data and/or the history of the sensed data.

In some embodiments, the corrective action taken by the controller varies depending on the determination of the blockage location. Optionally, a distal-tip blockage removal routine includes momentarily stopping evacuation. Simultaneous with the change in evacuation pressure, a flushing jet is delivered through the irrigation port, potentially knocking the blockage away from the tip. A potential advantage of this is more rapid cleaning, since already evacuated particles do not have to be regurgitated and evacuated a second time. A sufficiently interior blockage, however, is not affected by a flushing jet at the distal tip. Immediately making a reversal of flow direction in the evacuation channel is potentially preferred in such a case.

In some embodiments of the invention, sensor readings are used to make a determination of the severity of the blockage. For example, a partial blockage within the evacuation channel **203** may produce readings which are intermediate between those of readings **253** and **252**. In some embodiments, determination of an intermediate level of blockage is a potential determined status of the system. Optionally, the controller determines whether or not to begin a purging operation based on a threshold of blockage severity. In some embodiments, the threshold is user selectable, allowing a balance between speed of evacuation and sensitivity to potential blockage. A potential advantage of an adjustable threshold is the ability to adjust to different conditions of the bowel. For example, the evacuation phase of an evacuation/purge duty cycle may be set as long as possible for the condition of a given patient's colon without risking an unrecoverable blockage.

Another status determinable in some embodiments of the invention is the rate at which a blockage is developing. The determination by the controller to act or not on a partial blockage is optionally affected by the rate of increasing blockage. For example, a rapidly developing pressure change may be an indication of a developing hard blockage. Optionally, an incipient hard blockage is purged immediately even if the actual loss of flow is still low. In contrast, a slowly developing pressure change is optionally permitted to rise to a higher level of blockage. It may be, for example, associated with normal motions of the evacuation tube during insertion, or potentially determined to be most likely loose or self-correcting. Optionally, it is rapid fluctuations which have a lowered sensitivity, for example to filter out measurement noise and transients. Optionally, there are multiple categories of status determinable by controller **213**. In some embodiments, controller **213** ignores, for example, very fast small changes, acts quickly for somewhat slower or larger changes, and sets a high threshold for response to slow changes.

The use of pressure sensors in some embodiments of the invention allows determination of system status parameters optionally including-as the above examples describe-blockages and developing blockages, including their locations, severity, and/or rate of development. Use of a pressure sensor is typical for performing these and related sensing functions in some embodiments of the current invention. In some embodiments of the invention, other types of sensors and/or sensor systems are present on, near, or in the evacuation channel or the tip of the evacuation channel. These additional inputs are optionally comprised in further forms of status determination, and potentially affect system operation.

In some embodiments of the invention, the insertion tube comprises a sensor suitable for sensing the presence of fluid. A fluid sensor is, for example: a pH sensor, an osmotic pressure sensor, and/or an electrical conductance sensor. In some embodiments, the fluid sensor is disposed on the insertion tube of the colon cleaning system to detect fluid around the distal tip of the insertion tube. Optionally, this status determination automatically initiates a fluid evacuation routine to clear the fluid, with or without a simultaneous supply of irrigating fluid.

In some embodiments, an optical sensor senses material flow by means of optical flow near the sensor. According to the embodiment, an appropriate illumination source for the sensor is provided.

In some embodiments, the optical sensor reports spectral information, allowing determination of information about the composition of the waste material. For example, the presence of blood in the waste material is potentially indicated by waste material having the spectral characteristics of hemoglobin. A potential advantage of detecting blood in waste material is the detection of hemorrhaging, which is a possibly serious complication of a colonoscopy examination. Optional actions commanded by the controller in the case of a determined unexpected hemorrhage are cessation of vacuum to the colon, and/or raising an audible or visual alert.

In some embodiments of the invention, an optical sensor indicates to the controller the amount of waste content in evacuated fluid. Waste content is measured by, for example: optical density reflecting turbidity and/or solutes, particle counts, and/or particle size. In some embodiments of the invention, there is a threshold of indicated waste content below which fluid is considered clean. Optionally, an irrigating and evacuation routine is terminated when a threshold condition (for example, of cleanliness) is met, and/or when a threshold condition is met for a preset time period. Optionally, an irrigating and/or evacuation routine starts when a view through the colonoscope is determined to be obscured.

In some embodiments, the density, turbidity, and/or conductance of the fluid being pumped indicate the presence of foaming. Potentially, sensed indications of a need for purging, and optionally the parameters of purging itself are different when handling foamed compared to unfoamed evacuation material. For example, pressure changes indicative of a forming blockage are potentially different, due to the more compressible properties of a foam compared to a liquid. Also for example, the purging of a forming blockage in the presence of foamed material in some embodiments includes a lengthened purge cycle. Optionally, the lengthened cycle is long enough so that unfoamed material can be sent distally for more energetic purging action.

In some embodiments of the invention, a record of waste content amounts evacuated during a procedure is stored and made available online and/or for later retrieval. Optionally, retrieved data is in the form of a report. Optionally, reported waste content is as a function of time and/or position in the bowel. Optionally, waste content is reported as an integrated value. Using a reliable colon cleaning system during a colonoscopy, there is a potential to modify (for example, make less aggressive) protocols for cleaning a patient's bowel before examination. In some embodiments, cleaning can be done without one or more typical bowel cleaning steps, for example fasting or directed consumption of liquids. In some embodiments, operation is prepless-performed without advance colon cleaning steps. Feedback on the effectiveness of a particular pre-exam cleaning procedure is potentially beneficial for evaluating the effectiveness of alternative protocols. Additionally and/or alternatively, an objective indication during a procedure of unacceptably high waste content in a patient colon can be used to justify a corrective action and/or aborting the procedure.

In some embodiments of the invention, sensors and or determinations which indicate a status away from the evacuation channel are available to the controller **213**.

In some embodiments, sensing data and/or status indications are provided to the colon cleaning system from other systems or subsystems; for example: a colonoscope, or a clinical monitoring device. Optionally, these data and/or status indications directly trigger and/or halt a cleaning routine, or provide additional input to the controller **213** used in making operating determinations. Data and indications are communicated, for example, by a wire, optical coupling, radio, and/or another standard or specially provided communication method between the colon cleaning system and another system.

In some embodiments of the invention, for example, an automatically determined indication of the presence of obscuring material in the colonoscope view is optionally provided to controller **213**. Optionally, a cleaning routine is initiated upon receipt of such an indication.

### Exemplary Cleaning Routine

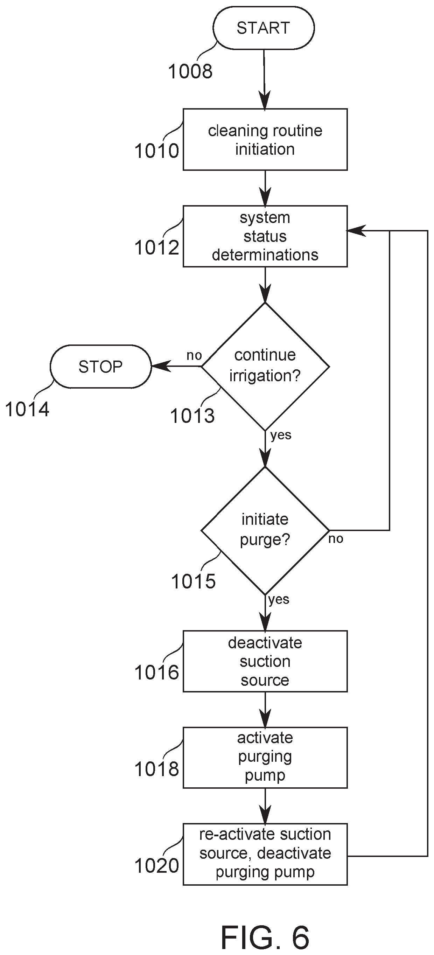

Reference is now made to *Figure 6*, which is a simplified flow chart of an exemplary method for cleaning a colon, including detection and purging of blockages, according to some exemplary embodiments of the invention. The method occurs, for example, in the course of an ongoing colonoscopy exam. Reference numbers not in *Figure 6* refer to earlier figures, for example *Figures 4A-4F*. In this exemplary method, pump **214.4** is treated as a vacuum source (moving material proximally), while pump **214.3** is treated as a pump for moving material distally in evacuation channel **203**.

At block **1008**, a determination to initiate a cleaning routine is made, according to some exemplary embodiments of the invention. The determination is made, for example, based on an elapsed time value, a manual command of a cleaning system operator, and/or determined system status. Optionally, for example, a determination of an immersion and/or an obscured view status initiates a cleaning routine.

At block **1010**, a cleaning routine initiates, according to some exemplary embodiments. Liquid is provided, for example by pump **214** through irrigation pipe **201**, to irrigate a colon. Suction is provided, for example, by pump **214.4** or another vacuum source through evacuation channel **203**. Suction moves fecal matter dissolved or suspended in the irrigation liquid from colon **1** proximally through channel **203**. Optionally, waste is collected in waste collection device **212A**. In some embodiments, the relative balance of suction and irrigation is under control by controller **213**. The balance is chosen, for example, to maintain an equal volume, or alternatively to provide net suction to clear an overpressure or fluid build-up. Optionally, the control is adjusted based on monitored sensor data.

At block **1012**, one or more system status determinations are made, according to some embodiments. Sensors and sensing modules, for example as described in relation to *Figures 2A-4F* or elsewhere herein, report pressure data and/or flow data to a controller **213**. The controller **213** makes one or more status determinations based on reported data. It should be noted that controller **213** is optionally implemented in a distributed fashion. For example, some sensing, status determination, and control may be integral to a pump (such sensing for a shutdown in a fault condition). Other status determination and control is optionally concentrated at a CPU, FPGA, or other logic circuit of the colon cleaning system.

At block **1013**, an irrigation continuation determination occurs, according to some embodiments. Controller **213** determines, for example, whether pressure safety limits are kept, whether a manual irrigation stop command has been issued, and/or whether a preset irrigation time has elapsed. If there is a determination to stop irrigation, irrigation terminates at block **1014** until next initiated.

At block **1014**, irrigation terminates, according to some embodiments.

At block **1015**, a purge initiation determination occurs, according to some embodiments. Controller **213** determines, for example, whether flow and/or pressure status is within parameters suitable for continuation of the cleaning process. A suitable pressure for initiation of a purge is, for example, a pressure which is determined to be within safe pressure limits for an intracolonic pressure. The safe pressure condition is, for example, a pressure less than 90-110 mmHg, less than 100-150 mmHg, less than 140-160 mmHg, or less than 150-200 mmHg. Additionally and/or alternatively, controller **213** determines if elapsed time of irrigation remains within a preset time period for irrigation before purging, and/or if no manual purging command has been issued. If there is a determination to purge, a purging routine activates at **1016**. Otherwise, cleaning continues at **1010**.

At block **1016**, the controller **213** commands the suction pumping source **214.4** to deactivate, according to some embodiments. Optionally, irrigation pump **214** is deactivated.

At block **1018**, the controller **213** commands the purging pump **214.3** to activate, according to some embodiments. Purging pump **214.3** sends water or another liquid from source **211** distally into channel **203**. Potentially, this dislodges the blockage. In some embodiments, purging continues until the determination of a blockage is cancelled, and/or until a preset time has elapsed. The preset time may be, for example, 50 msec, 100 msec, 200 msec, 500 msec, 1000 msec, any time in between, or a larger or smaller time. In some embodiments, a determination of a potentially dangerous pressure level in the colon stops the purging routine (deactivating purging pump **214.3**, for example). In some embodiments, the duration of the purging routine is determined by input from the operator of the device. In some embodiments, purging comprises a sequence of detailed purging actions and/or phases, as described hereinbelow.

At block **1020**, the suction pumping source **214.4** is reactivated, and the purging pump **214.3** is stopped. Cleaning continues at **1012**.

Details of the above method are optionally altered according to the embodiment of the invention:
In some embodiments of the invention, the role of pump **214.4** as a suction source is played, for example, by a regulated constant suction source. Such a source is, for example, a fixture in a hospital room connected to a central vacuum source, regulated through a valve. In some embodiments, pump **214.4** may thus alternatively be considered as valve **214.4**. Optionally, valve **214.4** is variable (for example, under control of controller **213**) to regulate the amount of suction to an amount appropriate to the determined status of the system.

In some embodiments, pump **214.4** is a bi-directional pump, driving proximal flow of matter in channel **203** during evacuating operation and driving distal flow of matter during purging operations. Purging pump **214.3** is optionally absent.

It is further noted that some individual features are shown, described, and explained with reference to specific exemplary embodiments and figures. However, it is to be understood that some embodiments of system **207** comprise combinations of features presented individually.

For example, figures presented herein illustrate sensors and sensing modules at various positions within embodiments of system **207**, and operating in various ways. Other figures herein present devices and methods by which an embodiment of system **207** undertakes one of various purging operations in response sensor data and user commands. Combinations of described sensors, status determinations, and actions taken in response, are considered embodiments of the invention.

### Irrigating and Evacuation Tip

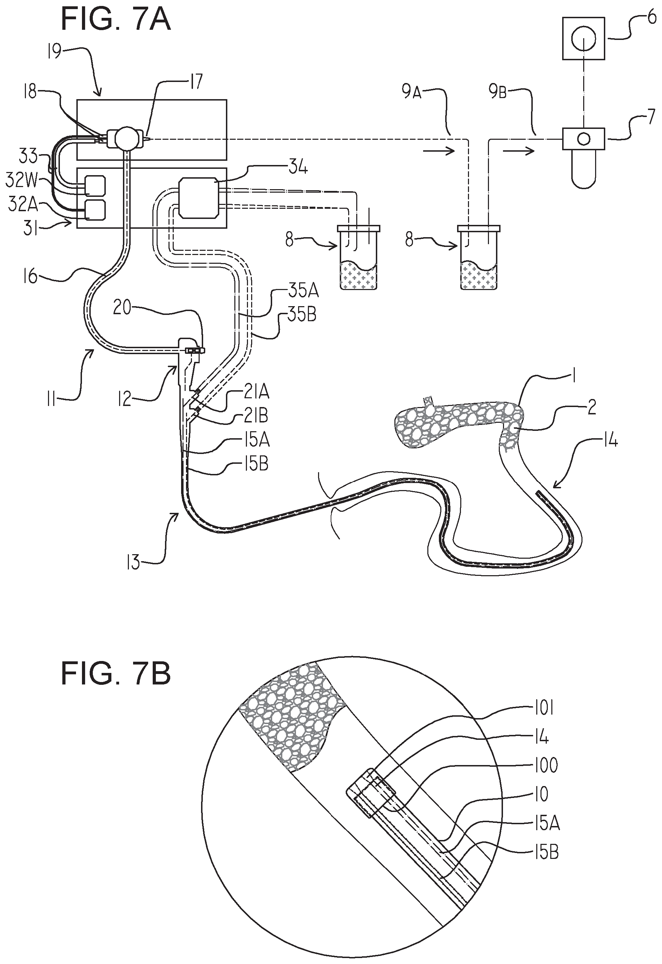

Reference is now made to *Figure 7B*, which schematically illustrates a tip **14** for use at the distal end of an insertion tube **13** of a colon cleansing system, according to some exemplary embodiments of the invention.

In some embodiments of the invention, tip **14** comprises an add-on adaptor element **100** at the distal end of the insertion tube of a colonoscope **10**. In some embodiments, adaptor element **100** comprises plurality of intake channels fed by intake apertures **101** in fluid communication with a corresponding plurality of colonoscope working channels **15A**, **15B**. In some embodiments, intake channel apertures **101** comprise a distally widening shape which tapers to the dimensions of working channels **15A**, **15B** at their mating point. A potential advantage of adaptor element **100** is to distribute suction exerted through channels **15A**, **15B** over a wider distal surface area. Potentially, fluid more easily enters the widened ends of channel intake apertures **101**. In some embodiments of the invention, tip **14** comprises a manifold, dividing an irrigation channel into 2, 3 4 or more outlets. Optionally, the outlets shape irrigation fluid into jets of fluid for more effective cleaning of material from the colon wall. In some embodiments, tip **14** comprises a pressure sensor for sampling pressure near the tip of the cleaning system and/or colonoscope.

Note in particular that the design of adaptor element **100** introduces the use of two or more channels for functions of a colon cleaning device. Additional details and potential advantages of a plurality of irrigating and/or flushing channels are provided hereinbelow.

### Colonoscope Cleaning Systems with Multiple Channels

Reference is now made to *Figure 7A*, which schematically illustrates a cleansing working station **31** wherein a plurality of irrigation and/or evacuation channels are provided, according to some exemplary embodiments of the invention.

In some embodiments, work station outlet pump **32** comprises pump **32A** for supplying gas and pump **32W** for supplying water.

In some embodiments of the invention, gas and water supply pumps **32A**, **32W** are operated simultaneously. In some embodiments, gas and water supplied under pressure are mixed (for example, in colonoscope workstation **19** or colonoscope workstation **31**) to be delivered through insertion tube **13**. In some embodiments, gas and water are mixed upon exiting their respective irrigation channels at tip **14**. Optionally, gas and water supply pumps **32A**, **32W** operate separately and/or alternately. The gas supplied by pump **32A** is, for example, air or carbon dioxide.

A potential advantage of supplying mixed gas and water for irrigation is to increase speed, thoroughness, and/or particle fragmentation during cleaning of colon **1**. Potentially, fecal material is loosened and/or broken apart by the time-varying forces exerted by vortices of alternating gas and water created within the mixed-phase irrigation material. Potentially, gas driven into crevices of fecal matter under compressive pressure expands when pressure releases, contributing to breakup of clumped fecal matter.

In some embodiments, evacuation pump **34** comprises a plurality of inlets for a plurality of evacuation pipes **35A**, **35B**. In some embodiments, colonoscope handle **12** comprises a plurality of work channel inlets **21A**, **21B** connected to the channels of evacuation pipes **35A**, **35B**. In some embodiments, the work channel inlets **21A**, **21B** themselves connect to a plurality of independent or largely independent working channels **15A**, **15B**. In some embodiments, working channels **15A**, **15B** are working channels of a colonoscope. In some embodiments, the plurality of evacuation channels is independently operable. In some embodiments, channel operation includes reversibility, for example to purge a blockage.

A potential advantage of a plurality of evacuation channels is the increase of lumenal cross-section through which evacuation occurs. Increased lumenal cross-section potentially increases the effectiveness of evacuation, for example, the rate of evacuation. Potentially, a single unblocked channel provides sufficient evacuation capacity, and the second channel is optionally reserved as a spare. Optionally, both channels are used together, but evacuation capacity remains even after the possible loss of one due to blockage during a procedure. Optionally, one channel is used to continue evacuation of waste while operations to purge a blockage in a second channel are carried out, for example, by reversing flow direction.

Another potential advantage of a plurality of evacuation channels is reduction of the maximum diameter of the tube **13** inserted into the colon, relative to its evacuation efficacy. For example, a single evacuation channel with a diameter of 5 mm potentially adds at least 5 mm to the maximum diameter of insertion tube **13**. The same cross-sectional area can be provided by, for example, two tubes of 3.5 mm diameter each, adding correspondingly less to the maximum cross-sectional of the insertion tube.

It should be noted that a non-circular shape is also possible. Potentially, however, a non-circular shape carries an increased risk of blockage for the same cross-sectional area. For example, the minimum dimension of a channel affects the maximum size of the smallest dimension of a particle which can pass through it. Also for example, a non-circular channel potentially increases regions of slow flow, which are in turn more vulnerable to blockages as a result.

According to the embodiment, the inner diameter of an evacuation channel is, for example, 2.1 mm, 3 mm, 4 mm, 4.2 mm, 4.5 mm, 5 mm, 5.5 mm, 6 mm, another larger or smaller diameter, or any diameter in between. According to the embodiment, where a plurality of channels is provided, the number of evacuation channels provided is, for example, 2, 3, 4 or more channels.

A potential advantage of a lowered diameter of an insertion tube is increased flexibility of the insertion tube **13**. Increased tube flexibility in turn potentially increases the probability of successfully navigating the tube through the colon to its end, so that the colonoscopy procedure can be completed successfully.

In some embodiments, channels are joined and/or merged along a portion of their length. For example, evacuation tubes **35A**, **35B** may be operated through a single input aperture of evacuation pump **34**. Also for example, working channels **15A**, **15B** may be joined at a point before reaching tip **14**, for example by original design, or by addition of an attachment (not shown). An optional point of joining near a proximal end of the channels may be, for example within 10 cm, 20 cm, 40 cm, 100 cm, any length in between, or a larger or smaller length of the proximal end. An optional point of joining near a distal end is within, for example, 1 cm, 2 cm, 4 cm, 10 cm, 20 cm, 40 cm, 100 cm, any length in between, or a larger or smaller length of the distal end.

A potential advantage of proximally joining evacuation tubes **35A**, **35B** over a portion of their length is reduced costs, for example of tubing, or of pump elements. Another potential advantage of joining or merging evacuation tubes **35A**, **35B** is the sharing of a larger diameter tube where practical. Optionally, this is in portions of the channel which are external to the colon during operation.

A potential advantage of distally connecting evacuation tubes **15A**, **15B** is the bypassing of blockages. In an example, two evacuation channels are each fed from the same two (shared) input apertures **101** of tip **14**. Thereby, a blockage in the body of one evacuation channel does not necessarily result in an otherwise unblocked tip aperture being rendered useless.

### Pluralities of Evacuation Channels

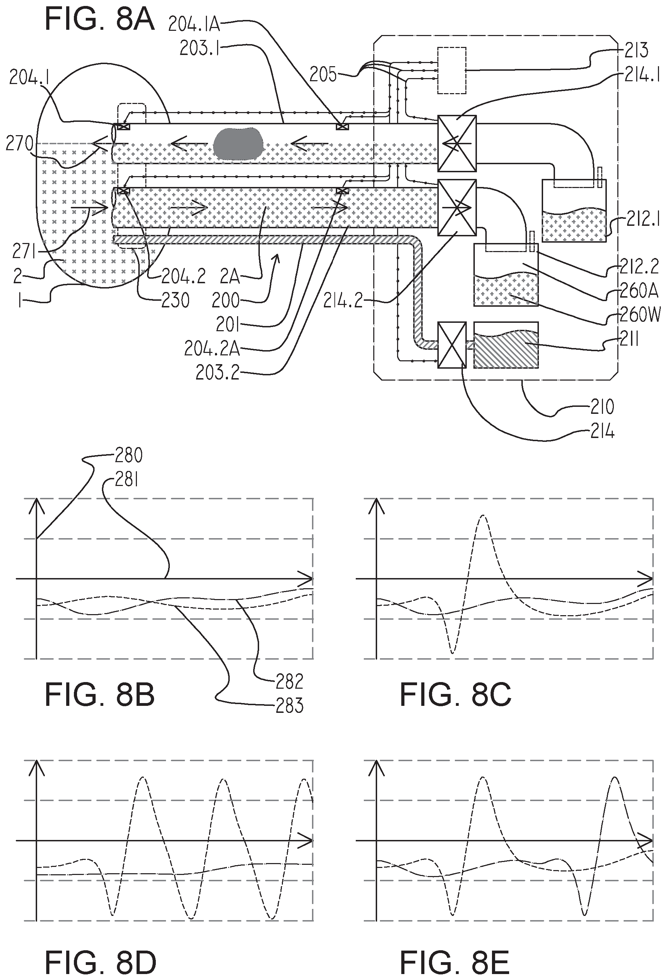

Reference is now made to *Figure 8A*, which schematically illustrates an intestine cleaning system comprising a plurality of evacuation channels, according to some embodiments of the present invention.

In some exemplary embodiments of the invention, two evacuation channels **203.1**, **203.2** interconnect the distal tip of a colon cleaning insertion tube with a proximally connected workstation. Optionally, more evacuation channels are provided.

A potential advantage of providing a plurality of evacuation channels is compactness in cross-section relative to a matched evacuation-capacity system with a single, larger-diameter evacuation channel. Another potential advantage of a plurality of evacuation channels is greater channel flexibility relative to a matched evacuation-capacity system with a single, larger evacuation channel.

It should be noted that an evacuation channel, in order to provide effective function, needs to remain open even under applied vacuum. For some embodiments of a colon cleaning device, this imposes interacting constraints which are met, according to the embodiment, by different combinations of design parameters. Particular considerations include:
- The choice of tube material, where increased rigidity potentially withstands an external pressure, but potentially interferes with flexibility.
- The tube lumen diameter, where lowered inner diameter potentially better withstands external pressure-particularly for a given wall thickness-but also decreases an upper size limit for fecal matter particles which can pass (there is also a potential trade-off with flexibility).
- The tube lumen shape, where a circular shape, for example, provides no inherent point of collapse, but limits the opportunity to distribute cross-sectional area close to the center of the insertion tube.
- The applied pressure gradient, where a higher gradient potentially allows faster evacuation through a given diameter of tubing, but increases a potential for tubing collapse, and may furthermore be limited in magnitude and/or time to avoid potential for injury.

Embodiments comprising a divided pair of circular lumens represent a functional compromise among these constraints (different from the compromise represented by a single lumen). In particular the minimum passage dimension remains large enough to pass particles of potentially blocking size, while the reduced overall diameter of the insertion tube retains flexibility and/or resists collapse. According to the embodiment, channel lumen diameter is, for example, 3 mm, 3.5 mm, 4 mm, 4.5 mm, 5 mm, 5.5 mm, 6 mm, any diameter in between, or another larger or smaller diameter.

Optionally, a more flexible material is usable with decreased lumenal cross-section. The material can be a softer silicone rubber or other material than that otherwise used in manufacture of a colonoscope or colon cleaning system insertion tube. The softness difference is a Shore A durometer decrease of, for example, 2 units, 4 units, 10 units, any value in between, or a larger or smaller unit value. Exemplary baseline Shore A durometer values for an insertion tube are between 50-70 Shore A durometer units. In some embodiments, a Shore A durometer of 40-50, or from 70-80 is used. It should be noted that pressurized fluid supplied to an insertion tube by a colon cleaning system potentially allows limited control of tube flexibility. Potentially, an inherently softer tube (when unpressurized) can nevertheless be stiffened by pressure as necessary during tube insertion.

In some embodiments, each evacuation channel **203.1**, **203.2** connects to a bi-directional pump **214.1**, **214.2** which both provides suction for moving material proximally in channel **203.1** or **203.2** and pumping action for moving material distally in the channels, as shown in *Figure 8A*.

This specific configuration is exemplary and not limiting. For example, in some embodiments, a plurality of channels **203.1**, **203.2** attaches to common vacuum sources, purge pumps **214.3**, and/or bi-directional pumps **214.1**, **214.2**. In some embodiments, each channel **203.1**, **203.2** individually connects both to an evacuation pump **214.4** (or to a connector for connecting to vacuum source outside the system) and also to a purge pump **214.3**.

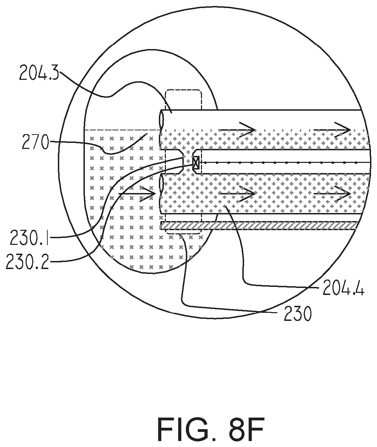

According to the embodiment, one or a plurality of irrigation pipes may be present. Where a plurality of irrigation pipes are provided, the pipes may be controlled individually or in combination. In the figure, irrigation pipe **201** operates to deliver irrigating fluids to a colon or other intestine portion near a distal end of evacuation channels **203.1**, **203.2**. Optionally, a bridging pipe (*Figure 8F* illustrates an example) connects between evacuation channels **203.1**, **203.2**, enabling fluid to cross between channels.

Some embodiments comprising a plurality of evacuation channels **203.1**, **203.2** are constructed, changed as necessary, as described herein with reference to embodiments comprising a single evacuation channel **203**. Methods and features described herein as embodiments of the present invention may also be implemented, changed as necessary, using a plurality of evacuation channels **203.1**, **203.2**. Optionally, channels operate individually (for example, with individually controlled pumps and/or individually controlled irrigation). Optionally, channels operate in tandem (for example, with pumping and irrigation from a common source and/or from individual sources controlled to behave similarly).

In some embodiments, evacuation channels **203.1**, **203.2** alternate between a first operating mode for evacuating irrigation and/or waste material proximally through a channel, and a second operating mode in which material is purged (pushed distally). Optionally, direction of flow is separately controlled for each channel **203.1**, **203.2** according to any of the methods for doing so described herein.

In some embodiments, controller **213** coordinates operating modes in two or more channels. For example, a system may be controlled so that when a first channel **203.1** is in a purge mode, a second channel **203.2** is in an evacuation mode. A potential advantage of operating channels simultaneously with inverted polarity is purging of blockage in one channel while continuing to move materials out of the intestine using another channel. Another potential advantage is reduced accumulation of material in the intestine during purging in a first channel, while a nearby second channel, in evacuation mode, simultaneously evacuates purging material. It is a potential advantage for positioning and/or collapse resistance to exert positive pressure in one channel for stiffening, while continuing to evacuate material reaching the colon interior. Another potential advantage is to alternately partially stiffen channels on different sides of an insertion tube, which potentially allows limited control over distal tip position within the colon.

In embodiments providing a distal fluid bridge between evacuation channels, operating a plurality of evacuation channels in opposite directions potentially prevents purged materials from reaching the intestine. For example, materials moved distally through a first channel **203.1** potentially cross the fluid bridge to be evacuated proximally by a second channel **203.2** supplied with suction.

### Operation of a Plurality of Evacuation Channels

Reference is now made to *Figures 8B-8E*, which are exemplary plots **282** (long-dashed), **283** (short-dashed) of relative pressures (Y-axis **280**) versus time (X-axis **281**) in a pair of evacuation channels **203.2**, **203.1** (respectively), according to some exemplary embodiments of the invention.

Pressures may be considered to be measured, for example, at the location of pressure sensors **204.1A**, **204.2A** inside evacuation channels **203.1** and **203.2**, respectively.

*Figure 8B* represents pressure readings under conditions of unblocked, ongoing evacuation. Pressure readings are negative, reflecting suction on the channels. Readings undergo relatively small fluctuations. Fluctuations are due to, for example, sensor noise, non-blocking particles in the system, mixtures of gas and fluid in the system, and/or movements of the insertion tube.

*Figure 8C* represents pressure readings during a period where a blockage occurs in and is removed from a single evacuation channel **203.1** (corresponding to short-dashed plot **283**). Initially, a pressure decrease is sensed at sensor **204.1A**, corresponding to an increasing degree of blockage. After the minimum of this decrease, the controller **213**, having determined that a blockage status exists, has sent a purging control signal to pump **214.1**. The resulting reversal of pressure from pump **214.1** is reflected in the subsequent rise of pressure to a high positive value.

In some embodiments of the invention, the reversal of pressure (which is comprised in a purge operation) is initiated contingent on a determination of another condition. In some embodiments, an intracolonic pressure safety condition must be met before reversal occurs. Optionally, a pressure safety condition with two available and functioning evacuation channels purged in alternation is less stringent than the condition when only one functioning evacuation channel is operated and/or when two functioning channels present a blocked state simultaneously. A pressure safety condition for initiation of a single channel purge is, for example, a pressure less than 90-110 mmHg, less than 100-150 mmHg, less than 140-160 mmHg, or less than 150-200 mmHg. Optionally, a pressure safety condition for initiation when two functioning channels are available for use in opposite directions of flow is, for example, 5 or 10 mmHg higher (but not higher than 200 mmHg). Optionally, a pressure safety condition for initiation when two functioning channels are simultaneously purged is, for example, 10 or 20 mmHg lower.

The pressure change that is determined to correspond to a blockage is, for example, 10 mbar, 20 mbar, 30 mbar, 40 mbar, 100 mbar, any pressure in between, or a larger or smaller pressure. Optionally, a minimum time is taken to determine that a pressure change corresponds to a blockage. This is, for example, 10 msec, 20 msec, 40 msec, 100 msec, 150 msec, 200 msec, 400 msec, any time in between, or a larger or smaller time. In some embodiments of the invention, the determination of a blockage is a combined function of time and pressure, for example, a change in pressure of 10-20 mbar over 10-20 msec, 20-50 msec, 50-100 msec, or 80-200 msec; a change in 20-50 mbar over 10-20 msec, 20-50 msec, 50-100 msec, or 80-200 msec; or a change in pressure of 80-120 mbar over 20-50 msec, 50-100 msec, or 100-200 msec. In some embodiments, the change is measured as a relative change between two sensor readings, for example a relative change of 10-20 mbar, 15-40 mbar, 30-80 mbar, or 75-100 mbar.

In some embodiments, the determination of a blockage status is according to a detected flow, a detected optically sensed condition, or according to another sensed condition as described herein.

According to the embodiment, the pressure increase of *Figure 8C* (represented by the plot peak) is maintained for a fixed maximum time. The time is, for example, 5 msec, 10 msec, 20 msec, 50 msec, 100 msec, 200 msec, any time in between, or a larger or smaller time. Optionally, the pressure peak is stopped when and/or maintained until a pressure change is sensed (for example, at sensor **204.1**), which is determined to correspond to a freeing of the blockage. Upon a determination to continue evacuation, controller **213** sends an evacuation control signal to pump **214.1**. Pump **214.1** reverses direction, pressure drops, and evacuation resumes.

In *Figure 8D*, a sequence of purging operations is shown, in which a blockage remains unresolved for at least two full purge/evacuation cycles. A purge sequence may occur, for example, if a timed purge cycle finishes, and the controller **213** determines that a blockage still exists. Alternatively, a predetermined purge routine may include a sequence of purge/evacuation cycles which are carried out without intermediate accounting for the blockage status of the system. The frequency of succeeding purge/evacuation cycles (a cycle being, for example, the period from peak evacuation pressure to purging and back to peak evacuation pressure) is, for example, 1 Hz, 2 Hz, 5 Hz, 10 Hz, 20 Hz, any frequency in between, or a larger or smaller frequency. The number of cycles in a train is, for example, 1 cycle, 2 cycles, 4 cycles, 10 cycles, 20 cycles, any number of in between, or a larger or smaller number. The number of purge trains occurring in a minute is, for example, 1-5, 3-8, 5-10, 8-20, or 15-30 distinct purging operations within a minute of system operating time. In some embodiments, the duty cycle of flow direction during purging (either distal/proximal or proximal/distal) is, for example 50% in each direction, 60%/40%, 70%/30%, 80%/20%, 90%/10%, or another duty cycle, as calculated for the portion of the overall duty cycle in which pressure is exerted. In some embodiments, there is a pause in pressure exertion comprising, for example, 5%, 10% or 20% of the overall duty cycle, during which no pressure is exerted.

In some embodiments of the invention, parameters of a purge cycle are adaptive. For example, the controller optionally tries different purging parameters during operation, and adjusts the preferred set of parameters used based on sensor readings during a purge program that indicate relative success in purging. In another example, a finding (for example, by the controller) during evacuation that trains of purge cycles are being triggered in a cluster is taken as an indication of a transient, blockage-forming clot in the system that remains a floating source of re-blockage even though it is repeatedly loosened. Optionally, an adaptive purge cycle adjustment is activated in such a situation to scrub the channel with a repeated train of cycles, prolonged beyond the determination that blockage is free, potentially breaking the problematic clot up before evacuation continues. Optionally, another purge program pumps fluid distally for a longer period than usual, in an attempt to push the blockage back out of the evacuation channel altogether.

In some embodiments of the invention, a purge cycle reduces a flow restriction in the evacuation lumen which comprises a cause other than or additional to a clot. For example, a flow restriction caused by a partial collapse of an evacuation channel under vacuum is reduced by lowering the proximally-directed pressure in the lumen of the evacuation channel. In another example, a kink in an evacuation lumen may be reduced by exerting distally-directed pressure, potentially forcing the kink to reopen. In another example, a turbulence arising in the evacuation channel, for example by exceeding a threshold of flow velocity compatible with laminar flow, may be reduced by changing the magnitude and/or direction of pressure in an evacuation channel.

In some embodiments of the invention, purging comprises variations in pressure in the same direction, optionally periodic. For example, a purging operation may continuously maintain a proximally-directed pressure, while increasing and decreasing it over time. Potentially, proximally-directed variation of pressure is sufficient to dislodge a weakly attached blockage, without a complete interruption in evacuation. In some embodiments, unidirectional pressure variations optionally alternate with variations of pressure in two directions. Potentially, this increases motion at the site of blockage (for example, as clot portions repeatedly flex to absorb changing pressure), contributing to freeing the blockage.

In *Figure 8E*, an alternating sequence of purging operations is shown, in which a blockage of one channel is immediately followed by a blockage in a second channel. The phases of evacuation, blockage, purging and return to evacuation are basically as described in connection, for example, to *Figure 8C*. There is an addition, in that channels operate separately. In some embodiments, purging in one channel is commanded together with increased evacuation in another, for example, to potentially maintain a net evacuation efficacy of fluid in the colon.

Reference is now made to *Figure 8F*, which illustrates an inter-evacuation channel fluid bridge **230.1**, according to some exemplary embodiments of the invention.

In some embodiments of the invention, a bridge **230.1** passable by fluid and/or waste is provided-for example, as part of a tip head attachment **230**-which creates a connection between two evacuation channels **204.3**, **204.4**. In some embodiments of the invention, a sensor **230.2** such as a pressure sensor is provided to monitor conditions within the bridge **230.1**.

In some embodiments, fluid bridge **230.1** plays a role in sensing if one of the intake apertures of the bridged evacuation channels **204.3**, **204.4** has become at least partially blocked. In some embodiments of the invention, the pressure differential resulting from a blockage diverts a portion of the fluid entering through the intake aperture of the unblocked channel aperture to divert through the bridge to the blocked channel. This produces a sensed change, for example, a pressure drop or a flow indication, which is optionally used as an indication of a blockage formation at the distal tip. In some embodiments of the invention, bridge **230.1** allows sharing by two or more channels of a single tip aperture, potentially permitting a larger portion of evacuation capacity to be maintained if one intake aperture becomes blocked during use.

### Disposable Pipe Apparatus

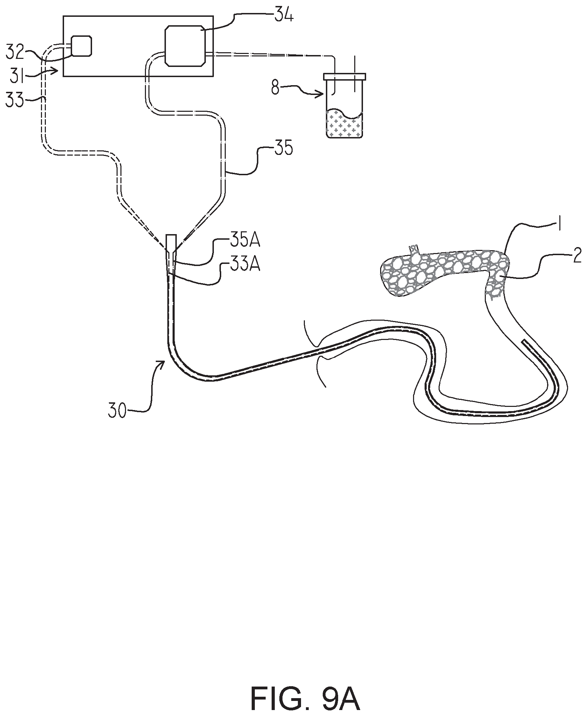

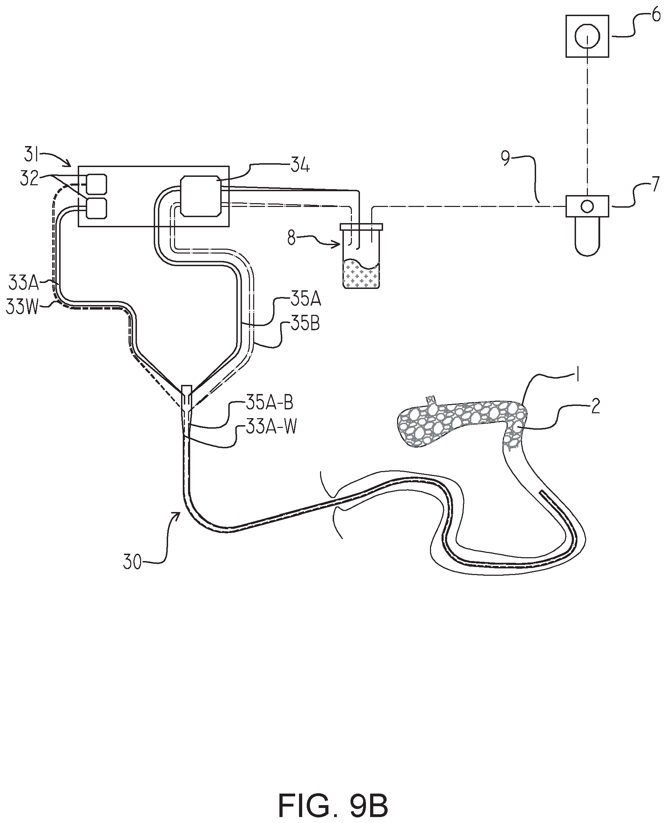

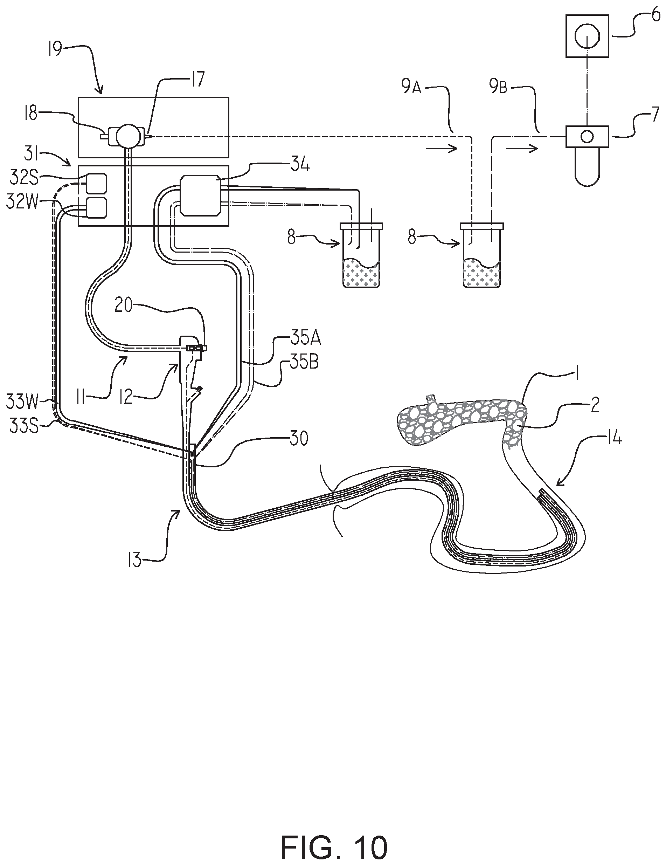

Reference is now made to *Figure 9A*, which schematically illustrates a cleansing workstation **31** with a colonoscope add-on tube assembly **30** comprising irrigation pipe **33A**, and evacuation pipe **35A**, according to some exemplary embodiments of the invention. Reference is also made to *Figure 9B*, which schematically illustrates an exemplary cleansing workstation **31** wherein disposable tube assembly **30** comprises gas and water pipes **33A**, **33W** for irrigation and evacuation pipes **35A**, **35B**. Reference is also made to *Figure 10*, which schematically illustrates an exemplary colonoscope workstation **19** operating adjacent to cleansing work station **31**.

In some embodiments of the invention, add-on tube assembly **30** is a tube for colon insertion. In some embodiments of the invention, tube assembly **30** is disposable. Optionally, the assembly is used for irrigation without a colonoscope.

A potential advantage of using a disposable tube assembly **30** for patient-contacting portions of a colonoscope cleaning system is to remove a need for cleaning and/or resterilization between uses. Another potential advantage of disposable tube assembly **30** is to allow rapid changeover in the event of an irreversible blockage in the disposable tube assembly **30** tubing.

In some embodiments of the invention, add-on tube assembly **30** is attachable to a colonoscope to be inserted into the colon **1** together with the colonoscope insertion tube **13**. In some embodiments, disposable tube assembly **30** comprises water tube pipe **33W** and gas tube pipe **33S** operating together for irrigation and/or sensing. In some embodiments, evacuation pipes **35A**, **35B** operate together and/or separately for evacuation.

A potential advantage of an add-on tube assembly **30** is to allow use of a colon cleaning system, for example, as a retrofit, with an existing colonoscope system. Another potential advantage of an add-on tube assembly is to keep existing channels of a colonoscope, for example, working channels, continuously free for use.

In some embodiments, pumps **32S**, **32W** supply different irrigation materials (for example, gas and water) via tubes **33W**, **33S** through disposable tube assembly **30** to colonoscope tip **14**. In some embodiments, pump **32S** creates a pressure used in remote pressure sensing, as described, for example, with reference to *Figures 3A-3E*. In some embodiments, pump **34** provides the pressure differentials which move irrigation material into fluids container **8** via tubes **35A**, **35B** through disposable tube assembly **30** from colonoscope tip **14**. In some embodiments, colonoscope tip **14** thereby supplies both irrigation material and irrigation material evacuation in the cleaning of fecal matter **2** from colon **1**.

In some embodiments of the invention, colonoscope work station **19** independently evacuates fecal matter. In some embodiments, a pressure differential for suction is provided by vacuum wall inlet **6** through regulator **7** via pipe **9**. In some embodiments, pipe **9** connects in turn through vacuum inlet **17** to colonoscope inner pipes **11**, insertion tube **13**, and tip **14**. Through this series of connections, fecal matter **2** is collectable by tip **14** into container **8**.

### Cleansing System with Disposable Tube Assembly

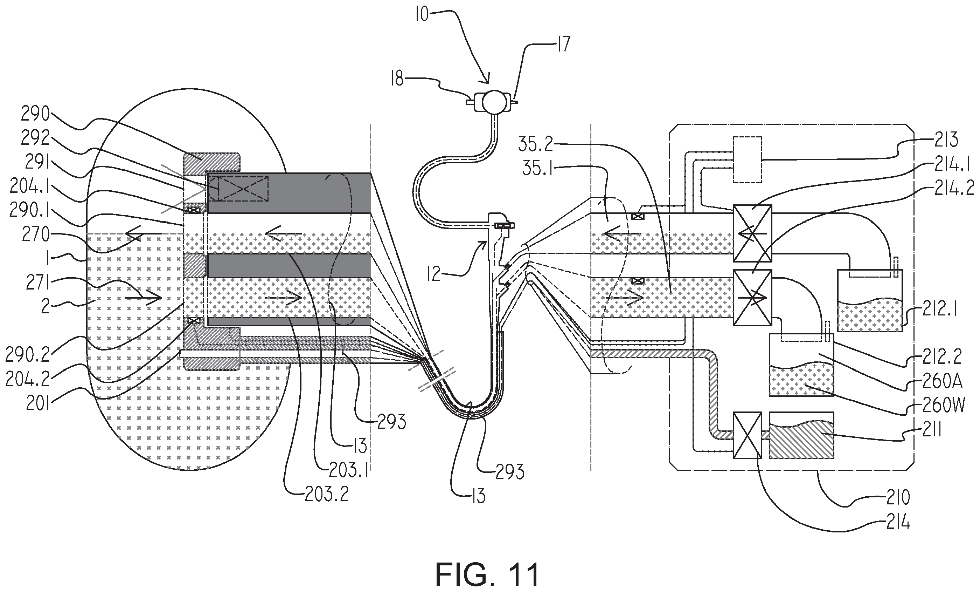

Reference is now made to *Figure 11*, which schematically illustrates an exemplary colon cleaning system comprising an add-on tube assembly and two bi-directional pumps together with a colonoscope, according to some exemplary embodiments of the invention.

In some embodiments of the invention, cleansing system **207** is attached to the probe of colonoscope **10**. In some embodiments, evacuation tubes **35.1**, **35.2** and irrigation tube **201** form disposable tube assembly **293**. The disposable tube assembly **293** and the colonoscope insertion tube **13** are assembled with a common distal head housing **290** at the tip. The disposable tube assembly **293** intubates colon **1** and evacuates fecal matter **2** along with insertion tube **13**. Suction **271** and evacuation **270** are controlled by work station **210** as described hereinabove.

In some embodiments of the invention, distal head housing **290** provides a framework for the mounting of sensors **204.2**, **204.1**, which attach to workstation **210** through tube assembly **293**. In some embodiments, distal head housing **290** comprises apertures for evacuation **290.1**, **290.2** which permit access to the working channels **203.1**, **203.2** of insertion tube **13**. In some embodiments, distal head housing **290** also provides an imaging aperture **291** for colonoscope imaging device **292** and/or associated illumination.

A potential advantage of using a head housing **290** is to allow add-on cleansing functionality together with an existing colonoscope. Some exemplary embodiments of the invention comprise a colonoscope originally designed to provide cleansing functionality as described herein, including purpose-provided irrigation and evacuation channels. In some embodiments, cleansing functionality is provided as an entirely separate tubing system, which does not interfere with the functions of a pre-existing colonoscope that it works alongside of. In some embodiments, such as that of *Figure 11*, the pre-existing colonoscope functionality is at least partially co-opted into cleaning. Optionally, one or more of the colonoscope working channels are used, at least part of the time, as evacuation channels for removing irrigation material from the colon. Optionally, functions such as irrigation and the directing of irrigation fluid are provided by one or more add-on units provided by the cleaning system. In such embodiments, head housing **290** optionally plays a role in integrating the structures of the colonoscope to the structures of the cleaning system. Furthermore, in some embodiments, with some portions of the tubing potentially usable either for cleansing or as working channels, the overall probe diameter can be reduced compared to that required for supporting each function independently.

As used herein the term "about" refers to within ±10%.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

The words "example" and "exemplary" are used herein to mean "serving as an example, instance or illustration". Any embodiment described as an "example or "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features except insofar as such features conflict.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

The disclosure of this application also includes the following numbered clauses:
Clause 1. A system for cleaning an intestine, comprising:
   an evacuation lumen for evacuating irrigation fluid from said intestine;
   a variable output pressure source;
   at least one sensor, positioned to detect an environmental condition in or near said evacuation lumen; and
   a controller configured to:
      determine a developing blockage of said evacuation lumen based on reporting from said at least one sensor, and
      vary the pressure from said pressure source for purging of said developing blockage based on said determination.
Clause 2. The system of clause 1, wherein said pressure source is operable to alternately apply proximally-directed and distally-directed pressure gradients to said evacuation lumen.
Clause 3. The system of any one of the preceding clauses, wherein said evacuation lumen is at least as long as a human colon.
Clause 4. The system of any one of the preceding clauses, wherein said developing blockage is determined while the rate of evacuation matches or exceeds the rate of introduction of said irrigation fluid to said intestine.
Clause 5. The system of any one of the preceding clauses, wherein said developing blockage comprises less than a 50% occlusion of a cross-section of an evacuation lumen.
Clause 6. The system of any one of the preceding clauses, wherein said developing blockage comprises less than a 10% occlusion of a cross-section of an evacuation lumen.
Clause 7. The system of any one of the preceding clauses, wherein said developing blockage comprises a decreasing rate of evacuation due to increasing obstruction of an evacuation lumen.
Clause 8. The system of any one of the preceding clauses, wherein said developing blockage comprises a blockage detected within 100 msec of reaching an occlusion of more than 50% of an evacuation lumen.
Clause 9. The system of any one of the preceding clauses, wherein said developing blockage comprises a blockage detected within 50 msec of reaching an occlusion of more than 80% of an evacuation lumen.
Clause 10. The system of any one of the preceding clauses, wherein said varying of pressure is configured to comprise at least two cycles of increasing and then decreasing a distally directed pressure.
Clause 11. The system of any one of the preceding clauses, wherein said controller is configured to redetermine blockage during said varying of pressure and adjust said varying of pressure based upon the finding of said redetermination being an unchanged blockage.
Clause 12. The system of any one of the preceding clauses, wherein said determination comprises determination of a location in the system of a developing blockage, said determination of a location being a basis for said variation of pressure.
Clause 13. The system of any one of the preceding clauses, wherein said at least one sensor comprises a plurality of sensors selected from a group consisting of:
   (a) a sensor positioned on external surface of a distal portion of said evacuation lumen and external to said lumen;
   (b) a sensor positioned within said evacuation lumen and within 5 mm of a distal end of said evacuation lumen;
   (c) a sensor positioned within said evacuation lumen and 5-30 mm of a distal end of said evacuation lumen;
   (d) a sensor positioned within said evacuation lumen and 3-160 cm from a distal end of said evacuation lumen;
   (e) a sensor positioned within said evacuation lumen and 160-250 cm from a distal end of said evacuation lumen;
   (f) a sensor positioned within said evacuation lumen and at least 250 cm from a distal end of said evacuation lumen; and
   (g) a sensor positioned within a fluid supply tube which supplies a fluid flow to a distal portion of said evacuation lumen.
Clause 14. The system of any one of the preceding clauses, wherein said pressure source comprises a pump operable to pump material distally through said evacuation lumen.
Clause 15. The system of clause 14, wherein said first pump is also operable to pump material proximally through said evacuation lumen.
Clause 16. The system of clause 14, wherein said pressure source comprises a second pump operable to pump material proximally through said evacuation lumen.
Clause 17. The system of clause 14, wherein said pressure source comprises a valve and vacuum source inlet, the valve being controllable for connecting and disconnecting said evacuation lumen with said vacuum source inlet.
Clause 18. The system of any one of the preceding clauses, wherein said pressure source comprises a valve and a pressurized fluid inlet, the valve being controllable for connecting and disconnecting said evacuation lumen with said pressurized fluid inlet.
Clause 19. The system of any one of the preceding clauses, wherein said at least one sensor comprises an optical sensor.
Clause 20. The system of clause 19, wherein said optical sensor senses at least one of the group consisting of a turbidity, a particle size, a particle number, or a particle spectrum.
Clause 21. The system of any one of the preceding clauses, wherein said at least one sensor comprises a sensor for sensing a bulk material property.
Clause 22. The system of clause 21, wherein said bulk material property is at least one of the group consisting of a pH, a conductance, a solute concentration, or an osmotic pressure.
Clause 23. The system of any one of the preceding clauses, wherein said at least one sensor comprises a flow sensor.
Clause 24. The system of any one of the preceding clauses, wherein said at least one sensor comprises a fluid pressure sensor.
Clause 25. The system of any one of the preceding clauses, wherein said determination of a developing blockage comprises at least one of the group consisting of a change in a sensed:
   (a) pressure from one or more sensors;
   (b) pressure difference among two or more sensors;
   (c) flow;
   (d) optical property; and
   (e) bulk material property.
Clause 26. The system of any one of the preceding clauses, wherein said system comprises a plurality of evacuation lumens.
Clause 27. The system of clause 26, wherein the pressure applied to each of said plurality of evacuation lumens by said pressure source is individually controllable.
Clause 28. The system of clause 27, wherein the evacuation pressure in a first of said plurality of evacuation lumens is distally directable, simultaneously with the evacuation pressure in a second of said plurality of evacuation lumens being proximally directed.
Clause 29. The system of clause 26, wherein said plurality of evacuation lumens comprises a joining connection within 100 cm of the distal end of said lumens, sized for the passage of evacuated material.
Clause 30. The system of any one of the preceding clauses, wherein:
   said at least one sensor is positioned to detect pressure in said evacuation lumen; and
   said determination of a developing blockage comprises determination of a pressure change in said evacuation lumen.
Clause 31. The system of clause 30, wherein said determination of a pressure change comprises determination of an onset of a pressure change, while said pressure continues to change.
Clause 32. The system of clause 30, wherein said determination of a pressure change comprises determination of a change within parameters selected from within the group consisting of:
   at least 10 mmHg change in pressure within 100 msec;
   at least 20 mmHg change in pressure within 150 msec;
   at least 30 mmHg change in pressure within 200 msec; and
   a differential change between two of said at least one sensor of at least 10 mmHg pressure within 100 msec.
Clause 33. The system of any one of the preceding clauses, comprising:
   an intestinal pressure sensor positioned to sense pressure within the intestine, outside of the evacuation lumen; and
   an irrigation supply channel supplying irrigation fluid to said intestine;
   said controller being configured to:
      determine an inflation pressure of said intestine based on reporting from said intestinal pressure sensor, and
      vary the supply of irrigation fluid to said intestine based on said determination.
Clause 34. A method for purging blockage of an evacuation lumen of an intestinal cleaning system, comprising:
   inserting an evacuation lumen at least 50 cm into a colon;
   evacuating waste from a colon proximally through said evacuation lumen;
   automatically detecting a developing blockage of the evacuation lumen; and
   initiating variation of the pressure in said evacuation lumen based on said automatic detection of a blockage.
Clause 35. The method of clause 34, further comprising:
   automatically determining that said developing blockage has been reduced; and
   stopping said automatic pressure variation.
Clause 36. The method of clause 34, wherein said pressure variation comprises alternating said pressure in said evacuation lumen between distally-directed and proximally-directed pressures.
Clause 37. The method of clause 36, wherein said pressure variation reverses the pressure between distally-directed and proximally-directed at a frequency above 1 Hz.
Clause 38. The method of clause 36, wherein said pressure variation reverses the pressure from distally-directed to proximally-directed at a frequency above 5 Hz.
Clause 39. The method of claim 34 wherein said automatic detection is performed at least 5 times per minute.
Clause 40. The method of clause 34, wherein said evacuation lumen comprises a plurality of evacuation sub-lumens arranged alongside each other, and said variation of pressure is controlled so that a distally-directed pressure is applied to one sub-lumen while a proximally-directed pressure is applied to another sub-lumen.
Clause 41. The method of clause 40, wherein said plurality of sub-lumens are joined by a connection allowing fluid communication, the join being within 20 mm of the distal end of said sub-lumens.
Clause 42. The method of clause 34, wherein said initiated variation of pressure is modulated based on at least one selected from the group consisting of:
   the determined degree of developing blockage;
   the determined location of developing blockage;
   a sensed reduction of flow in said evacuation lumen.
Clause 43. The method of clause 34 wherein said detecting of a developing blockage comprises a sensed pressure change in said evacuation lumen.
Clause 44. The method of clause 34 wherein the developing blockage detected is associated with a determined blockage location, and variation of pressure is modulated based on said blockage location.
Clause 45. The method of clause 44, further comprising changing a pressure of irrigation fluid supplied to the colon at the distal end of said evacuation lumen.
Clause 46. A system for cleaning an intestine, comprising:
   a plurality of evacuation lumens for evacuating irrigation fluid from said intestine;
   a pressure source operable to individually apply alternately proximally-directed and distally-directed pressure gradients to each of said plurality of evacuation lumens;
   at least one sensor, positioned to detect an environmental condition in or near said plurality of evacuation lumens;
   at least one of said at least one sensor being configured to report a level of intracolonic pressure; and
   a controller configured to:
      determine the presence of blockage of said evacuation lumen based on reporting from said at least one sensor,
      determine if the level of intracolonic pressure satisfies a pressure safety condition, and
      vary the pressure from said pressure source for purging of said blockage based on said blockage presence only if said pressure safety condition is met.
Clause 47. The system of claim 46, wherein said pressure safety condition is met by an intra-colonic pressure of less than 150 mmHg.
Clause 48. The system of claim 46, wherein said pressure safety condition is met by an intra-colonic pressure of less than 200 mmHg.
Clause 49. A system for cleaning an intestine, comprising:
   an evacuation lumen for evacuating irrigation fluid from said intestine;
   a pressure source operable to alternately apply proximally-directed and distally-directed pressure gradients to said evacuation lumen;
   at least one sensor, positioned to detect an environmental condition in or near said evacuation lumen; and
   a controller configured to:
      determine a flow restriction within said evacuation lumen based on reporting from said at least one sensor; and
      vary the pressure from said pressure source for reduction of said flow restriction based on said determination.
Clause 50. The system of clause 49, wherein said flow restriction is at least one selected from the group consisting of:
   a partial lining of material accumulated on the wall of said evacuation lumen;
   a change in the shape of said evacuation lumen;
   turbulence in flow through said evacuation lumen.

## Claims

1. A system for cleaning a body lumen and for purging a blockage in an evacuation lumen of said system, the system comprising:
an evacuation lumen for evacuating irrigation fluid from the body lumen as a flow of material;
a pressure source operable to cause a pressure gradient in said evacuation lumen by pumping material distally through said evacuation lumen or by pumping material proximally through said evacuation lumen;
at least one sensor, positioned to detect a pressure level or a change in pressure in or near said evacuation lumen, wherein a sampling rate of said at least one sensor is sufficient to detect intermediate pressure values between a blocked state of said evacuation lumen and an unblocked state of said evacuation lumen, said sampling rate being 5 Hz or higher; and
a controller configured to determine, based on the detection of said intermediate pressure values by said at least one sensor, a severity of the blockage in the evacuation lumen; said controller configured to control said pressure source, based on a threshold of the blockage severity, to reverse the pumping direction to thereby push said partial blockage and dislodge it from said evacuation lumen.

2. The system of claim 1, wherein said pressure source comprises a pump operable to alternately pump material distally through said evacuation lumen or proximally through said evacuation lumen.

3. The system of either one of the preceding claims, wherein said controller is configured to determine, based on the detection of said change in pressure by said at least one sensor, that said partial blockage is occluding less than 50% of a cross-section of the evacuation lumen.

4. The system of any one of the preceding claims, wherein said controller is configured to determine, based on the detection of said change in pressure by said at least one sensor, that said partial blockage is occluding less than 10% of a cross-section of the evacuation lumen.

5. The system of any one of the preceding claims, wherein said controller is configured to adjust said pressure gradient upon determining existence of said partial blockage by controlling said pressure source to increase and then decrease a distally-acting pressure produced by pumping of material distally through said evacuation lumen.

6. The system of any one of the preceding claims, wherein said controller is configured to determine a location in the system of the partial blockage, said location being a basis on which the pressure gradient is adjusted.

7. The system of any one of the preceding claims, wherein said at least one sensor comprises a plurality of sensors selected from a group consisting of:
(a) a sensor positioned on external surface of a distal portion of said evacuation lumen and external to said lumen;
(b) a sensor positioned within said evacuation lumen and within 5 mm of a distal end of said evacuation lumen;
(c) a sensor positioned within said evacuation lumen and 5-30 mm of a distal end of said evacuation lumen;
(d) a sensor positioned within said evacuation lumen and 3-160 cm from a distal end of said evacuation lumen;
(e) a sensor positioned within said evacuation lumen and 160-250 cm from a distal end of said evacuation lumen;
(f) a sensor positioned within said evacuation lumen and at least 250 cm from a distal end of said evacuation lumen; and
(g) a sensor positioned within a fluid supply tube which supplies a fluid flow to a distal portion of said evacuation lumen.

8. The system of any one of the preceding claims, wherein said at least one sensor comprises two sensors, and wherein said controller is configured to determine said partial blockage according to a pressure difference between said two sensors.

9. The system of any one of the preceding claims, wherein said system comprises a plurality of evacuation lumens; and the pressure gradient in each of said plurality of evacuation lumens is individually controllable by said pressure source.

10. The system of any one of the preceding claims, comprising:
a pressure sensor positioned to sense pressure within the body lumen, outside of the evacuation lumen; and
an irrigation supply channel supplying irrigation fluid to the body lumen;
said controller being configured to:
determine an inflation pressure of the body lumen based on reporting from said pressure sensor, and
vary the supply of irrigation fluid to the body lumen based on said determination.

11. The system of any one of the preceding claims, wherein said at least one sensor comprises a group of sensors.

12. The system of any one of the preceding claims, wherein a purging pulse applied by said pressure source to push said partial blockage and dislodge it is shorter than 500 msec.

13. The system of any one of the preceding claims, wherein said controller is configured to adjust a purging pulse applied by the pressure source based on the change in pressure detected by said at least one sensor.

14. The system of any one of the preceding claims, wherein the body lumen is one of the intestinal tract or specifically a colon.

15. The system of any one of the preceding claims, wherein said controller is configured to determine a combined function of a change in pressure within a particular time interval.
